(19) Europäisches Patentamt
European Patent Office
Office européen des brevets

(11) Publication number: 0 222 576 B1

(12) EUROPEAN PATENT SPECIFICATION

(45) Date of publication of patent specification: 18.03.92

(51) Int. Cl.[5]: C07D 417/06, C07D 413/14, C07D 413/06, C07D 403/06, C07D 498/04, C07D 417/12, C07D 409/06, C07D 513/04, A61K 31/50

(21) Application number: 86308545.2

(22) Date of filing: 03.11.86

# (54) Heterocyclic oxophthalazinyl acetic acids.

(30) Priority: 07.11.85 US 796039
07.11.85 US 796359

(43) Date of publication of application:
20.05.87 Bulletin 87/21

(45) Publication of the grant of the patent:
18.03.92 Bulletin 92/12

(84) Designated Contracting States:
AT BE CH DE ES FR GB GR IT LI LU NL SE

(56) References cited:
EP-A- 0 002 895

CHEMICAL ABSTRACTS, vol. 73, no. 5, 12th October 1970, page 366, abstract no. 77173y, Columbus, Ohio, US; S. FOLDEAK et al.: "Phthalazine and related heterocycles. X. Derivatives of 3-substituted 4-phthalazon-1-ylacetic acids", & KHIM.-FARM. ZH. 1970, 4(5), 22-6

(73) Proprietor: PFIZER INC.
235 East 42nd Street
New York, N.Y. 10017(US)

(72) Inventor: Mylari, Banavara Lakshmana
6, Ouinley Way
Waterford Connecticut(US)
Inventor: Larson, Eric Robert
88, Granite Road
Guilford Connecticut(US)
Inventor: Zembrowski, William James
1315, Route 163
Oakdale Connecticut(US)

(74) Representative: Wood, David John et al
PFIZER LIMITED, Ramsgate Road
Sandwich, Kent CT13 9NJ(GB)

EP 0 222 576 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to novel heterocyclic oxophthalazinyl acetic acids useful in the treatment of certain chronic complications arising from diabetes mellitus, such as diabetic cataracts, retinopathy and neuropathy, to pharmaceutical compositions containing such compounds and to a method of using these compounds.

In the past various attempts have been made to obtain more effective oral anti-diabetic agents. Generally these efforts have involved synthesis of new organic compounds, particularly sulfonyl ureas, and determination of their ability to substantially lower blood sugar levels when administered orally. However, little is known about the effect of organic compounds in preventing or alleviating chronic complications of diabetes, such as diabetic cataracts, neuropathy and retinopathy. U.S. Patent No. 3,821,383 discloses aldose reductase inhibitors like 1,3-dioxo-1H-benz-[d,e]-isoquinoline-2-(3H)-acetic acid and derivatives thereof to be useful for the treatment of these conditions. U.S. Patent 4,226,875 teaches the use of spiro-oxazolidinediones for treating complications of diabetes as aldose reductase inhibitors. Such aldose reductase inhibitors function by inhibiting the activity of the enzyme aldose reductase, which is primarily responsible for regulating the reduction of aldoses, such as glucose and galactose, to the corresponding polyols, such as sorbitol and galactitol, in humans and other animals. In this way unwanted accumulations of galactitol in the lens of galactosemic subjects and of sorbitol in the lens, peripheral nervous cord and kidneys of various diabetic subjects are prevented or reduced. Accordingly, such compounds are of therapeutic value as aldose reductase inhibitors for controlling certain chronic diabetic complications, including those of an ocular nature, since it is known in the art that the presence of polyols in the lens of the eye leads to cataract formation, with a concomitant loss of lens clarity.

U.S. Patent 4,251,528 discloses aromatic carbocyclic oxophthalazinyl acetic acids having aldose reductase inhibiting properties. The patent mentions that 2-(2-pyrid-2-ylethyl)-3,4-dihydro-4-oxophthalazin-1-ylacetic acid does not inhibit aldose reductase. Heterocyclic oxophthalazinyl acetic acids and their ethyl esters having an effect on the blood clotting system are disclosed in Chemical Abstracts 73, 77173y (1970).

According to the invention, compounds are provided having the formula

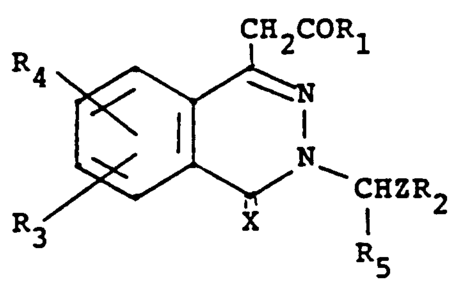

I

wherein

X is oxygen or sulfur;

Z is a covalent bond, O, S, NH or $CH_2$;

$R_1$ is hydroxy, or a prodrug group (as hereinafter defined); $R_2$ is a heterocyclic 5-membered ring having one nitrogen, oxygen or sulfur, two nitrogens one of which may be replaced by oxygen or sulfur, or three nitrogens one of which may be replaced by oxygen or sulfur, said ring substituted by one or two fluoro, chloro, $(C_1-C_4)$alkyl or phenyl, or condensed with benzo, or substituted by one of pyridyl, furyl or thienyl, said phenyl or benzo optionally substituted by one of iodo or trifluoromethylthio, or one or two of fluoro, chloro, bromo, $(C_1-C_4)$alkyl, $(C_1-C_4)$alkoxy, $(C_1-C_4)$alkylthio, $(C_1-C_4)$alkylsulfinyl, $(C_1-C_4)$alkylsulfonyl, or trifluoromethyl, and said pyridyl, furyl or thienyl optionally substituted in the 3-position by fluoro, chloro, bromo, $(C_1-C_4)$alkyl or $(C_1-C_4)$alkoxy; a heterocyclic 6-membered ring having one to three nitrogen atoms, or one or two nitrogen atoms and one oxygen or sulfur, and said ring substituted by one or two $(C_1-C_4)$alkyl or phenyl, or condensed with benzo, or substitued by one of pyridyl, furyl or thienyl, said phenyl or benzo optionally substituted by one of iodo or trifluoromethylthio, or one or two of fluoro, chloro, bromo, $(C_1-C_4)$-alkyl, $(C_1-C_4)$alkoxy, $(C_1-C_4)$alkylthio, $(C_1-C_4)$alkylsulfinyl, $(C_1-C_4)$alkylsulfonyl, or trifluoromethyl, and said pyridyl, furyl or thienyl optionally substituted in the 3-position by fluoro, chloro, $(C_1-C_4)$alkyl or $(C_1-C_4)$-alkoxy; oxazole or thiazole condensed with a 6-membered aromatic group containing one or two nitrogen atoms or with thiophene or furane, each optionally substituted by one of fluoro, chloro, bromo, trifluoromethyl, methylthio or methylsulfinyl; imidazolopyridine; naphthothiazole; or naphthoxazole; $R_3$ and $R_4$ are the same or different and are hydrogen, fluoro, chloro, bromo, trifluoromethyl, $(C_1-C_4)$alkyl, $(C_1-C_4)$-

alkoxy, ($C_1$-$C_4$)alkylthio, ($C_1$-$C_4$)alkylsulfinyl, ($C_1$-$C_4$)alkylsulfonyl, or nitro, or $R_3$ and $R_4$ taken together are ($C_1$-$C_4$)alkanedioxy; and $R_5$ is hydrogen or methyl; or a pharmaceutically acceptable base addition salt of a compound of formula I wherein $R_1$ is hydroxy, or an acid addition salt of a compound of formula I wherein prodrug group $R_1$ is di($C_1$-$C_4$)alkylamino or ($C_1$-$C_4$)alkoxy substituted by N-morpholino or di($C_1$-$C_4$)-alkylamino;

with the proviso that when $R_2$ is either a heterocyclic 5-membered ring having one heteroatom which is sulphur or a heterocyclic 6-membered ring having on heteroatom which is nitrogen, then said ring is either condensed with benzo, or substituted by one of pyridyl, furyl or thienyl, said benzo and said pyridyl, furyl and thienyl being optionally substituted as defined above.

Specific compounds of the invention are those wherein X is oxygen and those wherein $R_2$ is optionally substituted benzothiazolyl, benzoxazolyl, isoquinolyl, benzothiophen-yl, benzofuran-yl or benzimidazolyl, or substituted oxadiazolyl or indolyl.

Preferred compounds of the invention are those wherein X is oxygen, Z is a covalent bond or $CH_2$, $R_1$ is hydroxy, $R_2$ is optionally substituted benzothiazol-2-yl, benzothiazol-5-yl, benzoisothiazol-3-yl, benzoxazol-2-yl, 2-quinolyl, 2-quinoxalyl, oxazolo[4,5-b]pyridine-2-yl, benzothiophen-2-yl, benzofuran-2-yl, or thiazolo[4,5-b]pyridine-2-yl, or substituted 1,2,4-oxadiazol-3-yl, 1,2,4-oxadiazol-5-yl, isothiazol -5-yl, isothiazol-4-yl, 1,3,4-oxadiazol-5-yl, 1,2,5-thiadiazol-3-yl, oxazol-2-yl, thiazol-2-yl, or thiazol-4-yl, and $R_3$, $R_4$ and $R_5$ are hydrogen.

Other preferred compound are those wherein the methylene bridge connecting the oxophthalazinyl group with $R_2$ is located alpha with respect to a nitrogen atom in $R_2$, e.g. wherein $R_2$ is benzoxazol-2-yl or 1,2,4-oxadiazol-3-yl mentioned above.

Other specific compounds of the invention are those wherein $R_2$ is 2-benzothiazolyl substituted in the benzo by one trifluoromethylthio, or one or two of chloro, bromo, methyl, methoxy, trifluoromethyl, or 6,7-benzo, and those wherein $R_3$ is hydrogen, 5-fluoro, 5-chloro, 5-bromo or 5-methyl, and $R_4$ is hydrogen; 6-or 7- substituted chloro, bromo, methyl, isopropyl, methoxy, nitro or trifluoromethyl; 4,5-difluoro, or 5,7-dichloro; and those wherein $R_2$ is optionally substituted benzothiazol-2-yl or quinoxalyl and $R_3$ and $R_4$ are each chloro. Specific preferred compounds of formula I are 3-(5-bromo-2-benzothiazolylmethyl)-4-oxo-3H-phthalazin-1-yl-acetic acid, 3-(5-fluoro-2-benzothiazolylmethyl)-4-oxo-3H-phthalazin-1-ylacetic acid, 3-(5-trifluoromethyl-2-benzothiazolylmethyl)-4-oxo-3H-phthalazin-1-ylacetic acid, 3-(5-chloro-2-benzothiazolylmethyl)-4-oxo-3H-phthalazin-1-ylacetic acid, 3-(4,5-difluoro-2-benzothiazolylmethyl)-4-oxo-3H-phthalazin- 1-ylacetic acid, 3-(5,7-dichloro-2-benzothiazolylmethyl)-4-oxo-3H-phthalazin-1-ylacetic acid, and 3-(4,7-dichloro-2-benzothiazolylmethyl)-4-oxo-3H-phthalazin-1-ylacetic acid.

The present invention also relates to a composition for inhibition of aldose reductase activity comprising a compound of formula I in an amount effective in the inhibition of aldose reductase activity, in admixture with a pharmaceutically acceptable carrier. Specific and preferred compositions contain the specific and preferred compounds of formula I as described above.

The invention includes a process for preparing a compound of the formula I defined above by reacting a compound of the formula

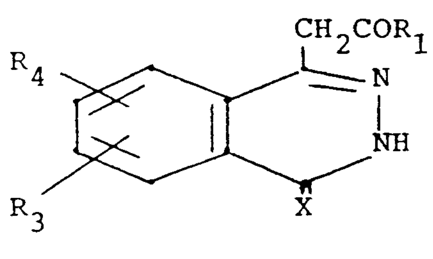

with a compound of the formula

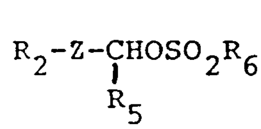

in an inert atmosphere, wherein $R_1$ is ($C_1$-$C_4$)alkoxy, X, Z, $R_2$, $R_3$, $R_4$ and $R_5$ are as defined in claim 1 and $R_6$ is ($C_1$-$C_4$)alkyl, trifluoromethyl, or phenyl optionally substituted by methyl, chloro, bromo or nitro.

The numbering system of the compounds of formula I is as shown:

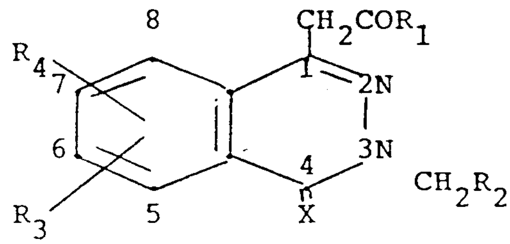

I

The term "($C_1$-$C_4$)alkyl" whenever used in the definitions of $R_1$ to $R_4$ denotes saturated monovalent straight or branched aliphatic hydrocarbon radicals having one to four carbon atoms, such as methyl, ethyl, propyl, butyl, t-butyl etc.

The term "prodrug" means a group that is converted in vivo into the active compound of formula I wherein $R_1$ is hydroxy. Such groups are generally known in the art and include ester forming groups, to form an ester prodrug, such as benzyloxy, di($C_1$-$C_4$)alkylaminoethyloxy, acetoxymethyl, pivaloyloxymethyl, phthalidoyl, ethoxycarbonyloxyethyl, 5-methyl-2-oxo-1,3-dioxol-4-yl methyl, and ($C_1$-$C_4$)alkoxy optionally substituted by N-morpholino and amide-forming groups such as di($C_1$-$C_4$)-alkylamino.

The heterocyclic 5-membered ring having one to three heteroatoms, one of which may be replaced by oxygen or sulfur includes imidazolyl, oxazolyl, thiazolyl, pyrazolyl, oxadiazolyl, thiadiazolyl, and triazolyl.

The heterocyclic 6-membered ring having one to three nitrogen atoms, or one or two nitrogen atoms and one oxygen or sulfur includes triazinyl, pyrimidyl, pyridazinyl, oxazinyl and triazinyl.

The heterocyclic ring may be condensed with benzo so that said ring is attached at two neighboring carbon atoms to form a phenyl group. Such benzoheterocyclic ring may be attached to Z either through the heterocyclic group or through the benzo group of the benzoheterocyclic ring. The preparation of those compounds wherein Z is attached to the benzo group is illustrated in Reaction Scheme B below. Specific examples wherein said heterocyclic ring is condensed with a benzo include benzoxazolyl, quinazolin-2-yl, 2-benzimidazolyl, quinazolin-4-yl and benzothiazolyl. The oxazole or thiazole condensed with a 6-membered aromatic group containing one or two nitrogen atoms include positional isomers such as oxazolo[4,5-b]-pyridine-2-yl, thiazolo[4,5-b]pyridine-2-yl, oxazolo[4,5-c]pyridine-2-yl, thiazolo[4,5-c]pyridine-2-yl, oxazolo-[5,4-b]pyridine-2-yl, thiazolo-[5,4-b]pyridine-2-yl, oxazolo[5,4-c]pyridine-2-yl, and thiazolo[5,4-c]pyridine-2-yl.

The compounds of the invention are prepared as outlined in Reaction Scheme A.

Phthalic anhydride and its derivatives of formula II are either commercially available or may be prepared according to standard procedures. The compounds of formula III wherein R' is ethyl or methyl may be prepared by reacting the compounds (II) with (carbethoxymethylene)triphenylphosphorane or (carbomethoxymethylene)triphenylphosphorane, respectively, in the Wittig reaction described in the prior art such as U.S. Patent 4,251,528 and Tetrahedron Letters, 1965, 2357.

The compounds of formula IV wherein R' is methyl or ethyl may be formed by reacting compounds (III) with hydrazine as described in U.S. Patent 4,251,528. Preferably, the reaction is carried out in an aqueous solvent such as aqueous ethanol, dioxane or dimethylformamide, and at 40° to 120°C, preferably, at reflux temperature.

The compounds of formula V are formed on reacting compounds (IV) wherein R' is hydrogen, methyl or ethyl, with

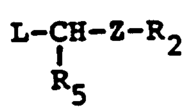

wherein L is a leaving group capable of forming compound LH on reaction of said two reagents. L is for example chloro, bromo, or $OSO_2R_6$, wherein $R_6$ is ($C_1$-$C_4$)alkyl, trifluoromethyl, phenyl or phenyl substituted by methyl, chloro, bromo or nitro.

## REACTION SCHEME A

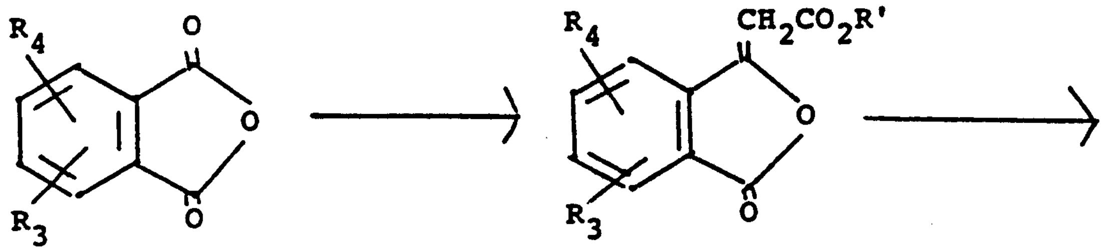

II III

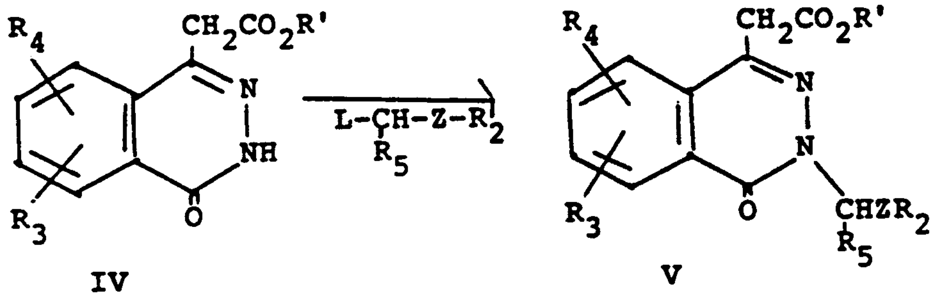


IV V

When reacting compounds (IV) wherein R' is methyl or ethyl, the process is generally carried out in a polar solvent such as an alkanol having 1 to 4 carbon atoms, e.g. methanol or ethanol, dioxan, dimethylformamide, or dimethylsulfoxide, in the presence of a base. Suitable bases are alkali metal hydride or alkoxide of 1 to 4 carbon atoms, such as sodium or potassium hydride, methoxide or ethoxide. When a hydride is used, a non-aqueous solvent such as dimethylformamide is required. When reacting compounds (IV) wherein R' is hydrogen, obtained on hydrolysis of compounds (IV) wherein R' is methyl or ethyl, it is necessary for at least two molar equivalents of the base to be present, since the first molar equivalent reacts with the carboxylic acid radical of such a compound. In addition, when reacting such compounds, it is preferable to use a hydroxylic solvent to minimize production of a corresponding ester.

The reaction to form compounds (V) wherein $R_5$ is alkyl is preferably performed with compounds of formula

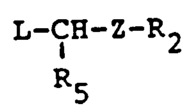

wherein L is $OSO_2R_6$ wherein $R_6$ is as defined above. This reaction is generally conducted in an inert atmosphere such as nitrogen in an aprotic polar solvent such as dimethylformamide at temperatures of 20 to 50°C.

The reaction to form compound (V) may be at room temperature, or at higher temperatures to accelerate the process.

The compounds of formula V wherein R' is methyl or ethyl may be hydrolyzed to obtain compounds of formula I wherein $R_1$ is hydrogen. The hydrolysis proceeds at conventional temperatures and in the presence of acid or base such as a mineral acid, for example hydrochloric acid, or an alkali metal hydroxide or carbonate such as sodium or potassium hydroxide or carbonate. The reaction is carried out in the presence of water and a solvent, for example an alkanol of 1 to 4 carbon atoms such as methanol, or dioxane.

The compounds of formula I wherein $R_1$ is hydroxyl may be esterified by conventional methods such as reaction of the corresponding acid chloride, bromide or anhydride with $R_1H$ to obtain compounds (I) wherein $R_1$ is an ester prodrug group. Alternatively, the compounds of formula I in which $R_1$ is an ester prodrug group may be prepared by alkylating a solution of the sodium salt of a compound (I) wherein $R_1$ is hydroxy. The alkylating agent may be a chloride. For instance, when $R_1$ is benzyloxy, acetoxymethyl, or pivaloyloxymethyl, then the alkylating agent is benzylchloride, chloromethylacetate or chloromethylpivalate, respectively. The above sodium salt is generally prepared in situ by reacting a compound (I) wherein $R_1$ is hydroxy with a sodium salt forming compound such as sodium bicarbonate, sodium hydride or sodium t-butylammonium sulfate in a non-aqueous solvent such as dimethylformamide or methylpyrrolidone.

When $R_1$ in compounds of formula (I) is an amide prodrug group such as di($C_1$-$C_4$)alkylamino, a compound (I) wherein $R_1$ is ($C_1$-$C_4$)alkoxy is converted to the corresponding amide by reaction with an amine, e.g. di($C_1$-$C_4$)alkylamine.

The compounds of formula I wherein X is sulfur are prepared by thiating the corresponding compounds (I) wherein X is oxygen by known procedures, for example by reaction with phosphorus pentasulphide.

The compounds of formula

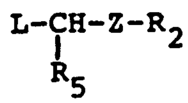

when L is chloro, Z is a covalent bond, $R_5$ is hydrogen, and $R_2$ is oxazole or thiazole condensed with Y wherein Y is a 6-membered aromatic group containing one or two nitrogen atoms, or with thiophene or with furane, each optionally substituted by one of fluoro, chloro, bromo, trifluoromethyl, methylthio or methylsulfinyl; or 1,2,4-oxadiazol-3-yl or 1,2,4-thiadiazol-3-yl optionally substituted by $R_7$ wherein $R_7$ is one of iodo or trifluoromethylthio, or one or two of fluoro, chloro, bromo, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl or trifluoromethyl, may be prepared by reacting compounds of the formula

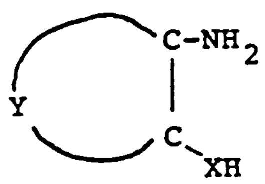

or

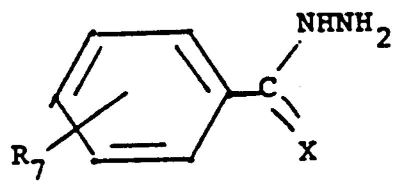

VI VII

wherein X is O or S, and Y and $R_7$ are as defined above, with tri(($C_1$-$C_4$)alkoxy)$CH_2Cl$. This reaction generally proceeds in a reaction-inert solvent such as a ($C_1$-$C_4$) alcohol e.g. ethanol, halocarbons e.g. chloroform or methylene chloride, or ethereal solvents such as diglyme. The reaction temperature ranges from about room temperature to the reflux temperature of the solvent used. The reaction time may range from about 15 minutes to about 2 hours or more.

The starting materials (VI) and (VII) are either commercially available or may be prepared according to standard procedures, e.g. as described in J. Am. Chem. Soc. 53, 309(1935) and J. Org. Chem. 29, 2652-(1964).

The tri(($C_1$-$C_4$)alkoxy)$CH_2Cl$ compounds are either known or may be prepared by reacting 1,1,1-trialkoxyethane with N-chlorosuccinimide, or with chlorine in pyridine and a chlorohydrocarbon solvent. The first chlorination reaction is generally carried out in a solvent, suitably a non-polar solvent such as carbontetrachloride or tetrachloroethylene. The reaction is conveniently carried out at temperatures ranging from about 40 °C to about the reflux temperature of the solvent. The reaction with chlorine in pyridine must be in the presence of a chlorohydrocarbon solvent having one or more chloro atoms and one to six carbon atoms, e.g. methylene chloride, chloroform or trichloroethane.

Reaction Scheme B exemplifies the preparation of 3-(benzothiazole-5-ylmethyl)-4-oxo-phthalazin-1-yl-acetic acid which is a compound wherein $R_2$ is a benzo-heterocyclic ring with the benzo attached to the methylene bridge in the final compound. Other such compounds wherein $R_2$ is a benzo-heterocyclic ring with the benzo attached to the Z in the final compound may be made by a similar method. In Scheme B, 5-methylbenzothiazole is reacted with a brominating agent such as N-bromosuccinimide to form 5-bromomethylbenzothiazole which is then reacted with 4-oxo-3H-phthalazin-1-yl acetate to form ethyl 3-(5-methylbenzothiazolyl)-4-oxo-3H-phthalazin-1-yl acetate under conditions as outlined above with reference to Reaction Scheme A for the conversion of compounds (IV) to compounds (V).

## REACTION SCHEME B

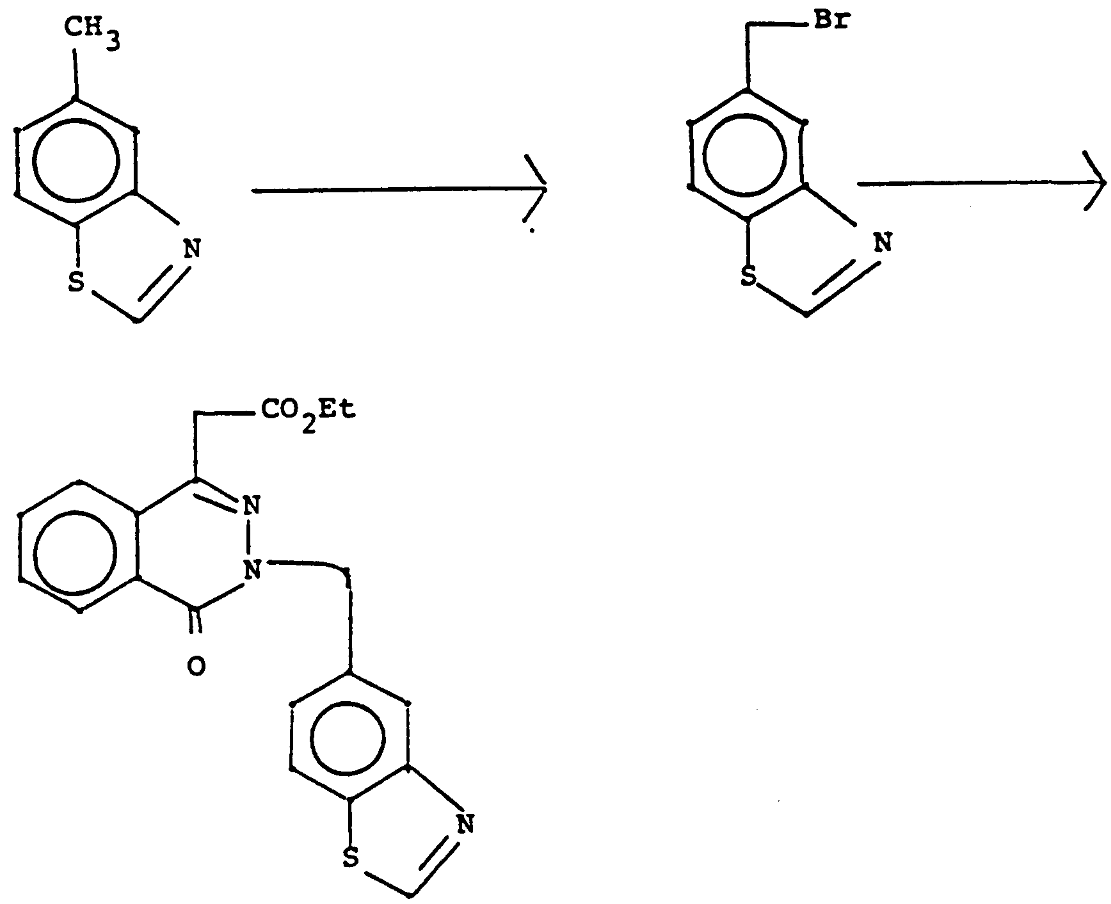

The pharmaceutically acceptable base addition salts of compounds (I) wherein $R_1$ is hydroxy may be formed with pharmaceutically acceptable cations by conventional methods. Thus, these salts may be readily prepared by treating the compound of formula I with an aqueous solution of the desired pharmaceutically acceptable cation and evaporating the resulting solution to dryness, preferably under reduced pressure. Alternatively, a lower alkyl alcohol solution of the compound of formula I may be mixed with an alkoxide of the desired metal and the solution subsequently evaporated to dryness. Suitable pharmaceutically acceptable cations for this purpose include, but are not limited to, alkali metal cations such as potassium and sodium, ammonium or water-soluble amine addition salts such as N-methylglucamine-(meglumine), the lower alkanolammonium and other base salts with organic amines which are pharmaceutically acceptable, and alkaline earth metal cations such as calcium and magnesium.

The pharmaceutically acceptable acid addition salts of the compounds of formula I are prepared in a conventional manner by treating a solution or suspension of the free base (I) with about one chemical equivalent of a pharmaceutically acceptable acid. Conventional concentration and recrystallization techniques are employed in isolating the salts. Illustrative of suitable acids are acetic, lactic, succinic, maleic, tartaric, citric, gluconic, ascorbic, benzoic, cinnamic, fumaric, sulfuric, phosphoric, hydroxhloric, hydrobromic, hydroiodic, sulfamic, sulfonic such as methanesulfonic, benzensulfonic, and related acids. Preferably, the acid is phosphoric acid.

The novel compounds of formula I and the pharmaceutically acceptable salts thereof are useful as inhibitors of the enzyme aldose reductase in the treatment of chronic complications of diabetes, such as diabetic cataracts, retinopathy and neuropathy. As used in the claims and specification hereof, treatment is meant to include both the prevention and alleviation of such conditions. The compound may be administered to a subject in need of treatment by a variety of conventional routes of administration, including orally, parenterally and topically. In general, these compounds will be administered orally or parenterally at dosages between about 0.5 and 25 mg./kg. body weight of the subject to be treated per day, preferably from about 1.0 to 10 mg./kg. However, some variation in dosage will necessarily occur depending on the

condition of the subject being treated. The person responsible for administration will, in any event, determine the appropriate dose for the individual subject.

The novel compound of the invention may be administered alone or in combination with pharmaceutically acceptable carriers, in either single or multiple doses. Suitable pharmaceutical carriers include inert solid diluents or fillers, sterile aqueous solution and various organic solvents. The pharmaceutical compositions formed by combining the novel compounds of formula I and the pharmaceutically acceptable carriers are then readily administered in a variety of dosage forms such as tablets, powders, lozenges, syrups and injectable solutions. These pharmaceutical compositions can, if desired, contain additional ingredients such as flavorings, binders and excipients. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the novel compound of formula I in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitioneal administration. In this connection, the sterile aqueous media employed are all readily available by standard techniques known to those skilled in the art.

Compounds of formula I may not only be advantageously employed for the preparation of aqueous pharmaceutical compositions for parenteral administration, as described above, but more particularly for the preparation of pharmaceutical compositions suitable for use as ophthalmic solutions. Such ophthalmic solutions are of principal interest for the treatment of diabetic cataracts by topical administration and the treatment of such conditions in this manner is a preferred embodiment of the present invention. Thus, for the treatment of diabetic cataracts the compounds of this invention are administered to the eye of an ophthalmic preparation prepared in accordance with conventional pharmaceutical practice, see for example "Remington's Pharmaceutical Sciences" 15th Edition, pages 1488 to 1501 (Mack Publishing Co., Easton, Pa). The ophthalmic preparation will contain a compound of formula I or a pharmaceutically acceptable salt thereof in a concentration from about 0.01 to about 1% by weight, preferably from about 0.05 to about 0.5% in a pharmaceutically acceptable solution, suspension or ointment. Some variation in concentration will necessarily occur, depending on the particular compound employed, the condition of the subject to be treated and the like, and the person responsible for treatment will determine the most suitable concentration for the individual subject. The ophthalmic preparation will preferably be in the form of a sterile aqueous solution containing, if desired, additional ingredients, for example preservatives, buffers, tonicity agents, antioxidants and stabilizers, nonionic wetting or clarifying agents and viscosity-increasing agents. Suitable preservatives include benzalkonium chloride, benzethonium chloride, chlorobutanol and thimerosal. Suitable buffers include boric acid, sodium and potassium bicarbonate, sodium and potassium borate, sodium and potassium carbonate, sodium acetate and sodium biphosphate, in amounts sufficient to maintain the pH at between about 6 to 8, preferably between about 7 and 7.5. Suitable tonicity agents are dextran 40, dextran 70, dextrose, glycerin, potassium chloride, propylene glycol and sodium chloride, such that the sodium chloride equivalent of the ophthalmic solution is in the range 0.9 plus or minus 0.2%. Suitable antioxidants and stabilizers include sodium bisulfite, sodium metabisulfite, sodium thiosulfite and thiourea. Suitable wetting and clarifying agents include polysorbate 80, polysorbate 20, poloxamer 282 and tyloxapol. Suitable viscosity-increasing agents include dextran 40, dextran 70, gelatin, glycerin, hydroxyethylcellulose, hydroxmethylpropylcellulose, lanolin, methylcellulose, petrolatum, polyethylene glycol, polyvinyl alcohol, polyvinylpyrrolidone and carboxymethylcellulose. The ophthalmic preparation will be administered topically to the eye of the subject in need of treatment by conventional methods, for example in the form of drops or by bathing the eye in the ophthalmic solution.

The activity of the compounds of the present invention as agents for the control of chronic diabetic complications may be determined by a number of standard biological or pharmacological tests. Suitable tests include (1) measuring their ability to inhibit the enzyme activity of isolated aldose reductase; (2) measuring their ability to reduce or inhibit sorbitol accumulation in the sciatic nerve and lens of acutely

streptozotocinized, i.e. diabetic, rats; (3) measuring their ability to reverse already-elevated sorbitol levels in the sciatic nerve and lens of chronic streptozotocin-induced diabetic rats; (4) measuring their ability to prevent or inhibit galactitol formation in the lens of acutely galactosemic rats; (5) measuring their ability to delay cataract formation and reduce the severity of lens opacities in chronic galactosemic rats; (6) measuring their ability to prevent sorbitol accumulation and cataract formation in isolated rat lens inclubated with glucose; and (7) measuring their ability to reduce already elevated sorbitol levels in isolated rat lens incubated with glucose.

The present invention is illustrated by the following examples. Proton nuclear magnetic resonance spectra (ʹHNMR) were measured for solutions in deuterochoroform ($CDCl_3$) and peak positions are expressed in parts per million (ppm) downfield from tetramethylsilane (TMS). The peak shapes are denoted as follows: s, singlet; d, doublet; t, triplet; q, quartet; m, multiplet; br, broad.

EXAMPLE 1

3-(5-Bromobenzothiazol-2-yl-methyl)4-oxo-3H-phthalazin-1-ylacetic acid (I; $R_1$ = OH; $R_3$ = $R_4$ = H; $R_2$ = 5-bromobenzothiazol-2-yl; X = O).

A. 2,5-Dibromothioacetanilide

A mixture of 2,5-dibromoacetanilide (45.0 g), phophorous pentasulfide (24.4 g) and benzene (500 ml) was refluxed for 18 hours. After cooling the reaction mixture, the benzene layer was decanted off and then extracted with 10% potassium hydroxide solution (2 × 75 ml. The basic aqueous extract was washed with ether (2 × 50 ml), and acidified to pH 4.0 by the addition of diluted hydrochloric acid, and the precipitated 2,5-dibromothioacetanilide was collected and then air dried (Yield: 14.4 g; m.p. 119-124° C).

B. 2-Methyl-5-bromobenzothiazole

This compound was prepared by adapting the procedure described in Synthesis, 1976, 731. To a solution of 2,5-dibromothioacetanilide (9.27 g) in N-methyl-pyrrolidinone was cautiously added sodium hydride (1.93 g as 50% w/w dispersion in mineral oil). After the addition was complete, the mixture was heated at 150° C for 1.5 hours. The dark reaction mixture was poured on to ice-water (300 ml.) and the separated brown gum was extracted with ethyl acetate (2 × 100 ml). The organic extract was washed with water (2 × 100 ml), dried over anhydrous magnesium sulfate and then evaporated. The resulting crude solid was chromatographed over silica gel to obtain the title compound (4.7 g; m.p. 84-85° C.).

C. 2-Bromomethyl-5-bromobenzothiazole

A mixture of 2-methyl-5-bromobenzothiazole (32.0 g), N-bromosuccinimide (25.1 g), carbon tetrachloride (700 ml) and a catalytic amount of benzoyl peroxide (0.2 g) was refluxed under irradiation by an UV lamp for 14 hours. The reaction mixture was cooled to room temperature, filtered to remove the precipitated succinimide and the filtrate was evaporated to dryness. The resulting solid was chromatographed over silica gel to obtain the product (7.8 g; m.p. 107° C).

D. Ethyl 3-(5-bromobenzothiazol-2-ylmethyl)-4-oxo-3H-phthalazin-1-ylacetate

To a mixture of ethyl 4-oxo-3H-phthalazin-1-ylacetate (11.6 g.) and sodium hydride (288 g; 50% w/w dispersion in mineral oil) in dimethylformamide (150 ml) was added 5-bromo-2-bromomethylbenzothiazole (16.8 g) and the resulting mixture stirred at room temperature for 1 hour. This reaction mixture was poured over ice-water (500 ml.); sufficient 10% HCl was added to adjust the pH to about 4.0 and the precipitated crude solid was collected. This was chromatographed over silica gel to obtain the product (yield: 15.6 g; m.p. 160-161° C).

E. 3-(5-Bromobenzothiazole-2-ylmethyl)-4-oxo-3H-phthalazin-1-ylacetic acid

A mixture of ethyl 3-(5-bromobenzothiazol-2ylmethyl)-4-oxo-3-H-phthalazin-1-ylacetate (15.0 g) and dioxane (150 ml) was brought to solution by warming on a steam bath and to this solution was added a solution of 10% potassium hydroxide (20 ml) in ethanol (50 ml.). The resulting dark purple solution was stirred at room temperature for 2 hours and concentrated to remove excess dioxane and ethanol. The

concentrate was diluted with water (100 ml) and the resulting solution was washed with ether (2 × 100 ml). The aqueous layer was collected and acidified to pH 2.0 by addition of concentrated HCl. The precipitated solid was crystallized from methylene chloride/ethanol (400 ml/40 ml) to obtain the product (Yield 7.65 g; m.p. 214 °C).

EXAMPLE 2

3-(2-Pyridyl-1,2,4-oxadiazol-5-ylmethyl)-4-oxo-3H-phthalazin-1-ylaetic acid (I; $R_1$ = OH; $R_3$ = $R_4$ = H; $R_2$ = 2-pyridyl-1,2,4-oxadiazol-5-yl; X = O)

A. Pyridamidoxime

A mixture of 2-cyanopyridine (15 g), hydroxylamine hydrochloride (10 g), sodium carbonate (15.3 g) and ethanol (100 ml) was refluxed for 24 hours. The reaction mixture was cooled, filtered and the filtrate evaporated to obtain a solid, which was extracted with ethyl acetate. The organic extract was dried, evaporated and the residue crystallized from benzene (3.0 g; m.p. 113-114 °C).

B. 3-(2-Pyridyl)-5-chloromethyl-1,2-4-oxadiazole

A mixture of pyridamidoxime (4.5 g), chloracetic anhydride (8.4 g) and toluene (350 ml) was refluxed for 12 hours. The hot solution was cooled, washed with water (2 × 100 ml), saturated bicarbonate solution (2 × 50 ml), again with water (2 × 100 ml) and the organic layer was evaporated to obtain a crude solid. This solid was crystallized from hexane to yield the product (3.4 g; m.p. 89-94 °C).

C. Ethyl 3-(2-pyridyl-1,2,4-oxadiazol-5-ylmethyl)-4-oxo-3H-phthalazin-1-ylacetate

To a solution of ethyl 4-oxo-3H-phthalazin-1-ylacetate (1.6 g) and sodium hydride (0.5 g; 50% w/w dispersion in mineral oil) in dimethylformamide (15 ml) was added 3-(2-pyridyl)-5-chloromethyl-1,2,4-oxadiazole (1.5 g) dropwise over a period of 40 minutes. After stirring for another 10 minutes, the reaction mixture was poured onto water (50 ml) and extracted with ether. The ether layer was evaporated and the residue was chromatographed over silica gel to obtain the product (1.0 g; m.p. 118-128 °C).

D. 3-(2-Pyridyl-1,2,4-oxadiazol-5-ylmethyl)-4-oxo-3H-phthalazin-1-ylaetic acid

To a solution of ethyl 3(2-pyridyl-1,2,4-oxadiazol-5-ylmethyl)-4-oxo-3H-phthalazin-1-ylacetate (1.0 g) in methanol (10 ml) was added 20% aqueous KOH (0.5 ml) and the mixture refluxed for 30 minutes. Evaporation of excess methanol gave an orange residue. The residue was dissolved in water, acidified with acetic acid (1 ml) and the precipitated solid was collected and crystallized from isopropanol to yield the product (0.53 g; m.p. 196-200 °C).

EXAMPLE 3

3-[3-(2-Trifluoromethylphenyl)-1,2,4-oxadiazol-2-ylmethyl]-4-oxo-3 -phthalazin-1-ylacetic acid (I; $R_1$ = OH; $R_3$ = $R_4$ = H; $R_2$ = 3-(2-trifluoromethylphenyl)-1,2,4-oxadiazol-2-yl)

A. 3-[2-Trifluoromethylphenyl]-5-chloromethyl-2,3,4-oxadiazole

2-Trifluoromethylbenzimidioxime prepared similary to the procedure described in Ber, 1899, 32, (1975) (2.9 g; m.p. 115-116 °C) starting from 2-trifluoromethylbenzaldehyde, was dissolved in anhydrous acetone (70 ml) and then solid potassium carbonate (2.0 g) was added. To the resulting slurry cooled to 15-18 °C in an ice-water bath was added a solution of chloroacetyl chloride (1.1 ml) dissolved in acetone (10 ml). After the addition, the ice-bath was removed and the reaction mixture brought to room temperature and stirred for 1.5 hours. Evaporation of acetone gave a white residue which upon trituration with water yielded 0-chloroacetyl-2-trifluoromethylbenzimidoxine (3.0 g; m.p. 108-110 °C). This product was mixed with tolune (50 ml) and heated to reflux for 1.5 hours. The toluene solution was cooled, washed with saturated aqueous sodium bicarbonate (10 ml) and water, and the organic portion was dried and evaporated. The resulting brown oil was chromatographed over silica gel to obtain the title compound as a yellow oil. ′HNMR($CDCl_3$, 60MHz): 4.0 (s, 2H), 4.4 (s, 2H), 7.3 (s, 5H).

B. Ethyl 3-[3-(2-trifluoromethylphenyl)-1,2,4-oxadiazol-2-ylmethyl]-4-oxo-3H-phthalazin-1-ylacetate

To a mixture of 4-oxo-3H-phthalazin-1-ylacetate (1.4 g) and sodium hydride (0.43 g; 50% w/w dispersion in mineral oil) in dimethylformamide (10 ml) was added 3-(2-trifluoromethylphenyl)-5-chloromethyl-1,2,4-oxadiazole (1.7 g) and stirred at room temperature for 30 minutes. The reaction mixture was poured onto water (20 ml), acidified to pH 2.0 with 10% HCl and the precipitated solid collected. The solid was triturated with isopropanol to obtain the product as a white crystalline solid (1.65 g; m.p. 111-115 ° C).

C. 3-[3-(2-Trifluoromethylphenyl)-1,2,4-oxadiazol-2-ylmethyl]-4-oxo-3 -phthalazin-1-ylacetic acid

A mixture of ethyl-3-[2-trifluoromethylphenyl)-1,2,4-oxadiazol-2-yl-methyl-4-oxo-phthalazin-1-ylacetate (1.6 g) and methanol (50 ml) containing 20 w/w aqueous KOH (0.5 ml) was heated on a steambath for 1 hour. Water (20 ml) was added to the residue obtained upon evaporation of methanol and the pH of the solution brought to 2 by addition of 10% HCl. The precipitated solid was crystallized from benzene (0.7 g; m.p. 132-134 ° C).

EXAMPLE 4

3-(N-methylbenzimidazol-2-ylmethyl)-4-oxo-3H-phthalazin-1-ylacetic acid (I; $R_1$ = OH; $R_3$ = $R_4$ = H; X = 0; $R_2$ = 3-(N-methylbenzimidazol-2-yl))

A. Ethyl 3-(N-methylbenzimidazol-2-ylmethyl)-4-oxo-3H-phthalazin-1-ylacetate)

A mixture of ethyl 4-oxo-3H-phthalazin-1-ylacetate (2.34 g) and sodium hydride (0.58 g; 50% w/w dispersion in mineral oil) in dimethylformamide (20 ml) was stirred at room temperature for 15 minutes. To this was added N-methyl-2-chlolomethylbenzimidazole (2.4 g; prepared according to JACS, 1943, 65 (1854) and stirred for another hour. It was poured onto water (150 ml) and extracted with ethyl acetate (2 × 100 ml). The organic extract was dried, evaporated and the residue was chromatographed over silica gel to obtain the product (2.04 g; m.p. 118 ° C).

B. 3-(N-methylbenzimidazol-2-ylmethyl)-4-oxo-3H-phthalazin-1-ylacetic acid

To a solution of 3-(N-methylbenzimidazol-2-yl)-4-oxo-3H-phthalazin-1-ylacetate (2.0 g) in warm methanol (100 ml) was added 10% KOH (10 ml), stirred at room temperature for 2.5 hours and then evaporated to obtain a solid. This solid was dissolved in water (50 ml), extracted with ether (50 ml) and the aqueous layer was adjusted to pH 6.0 by the addition of acetic acid. The resulting white solid was collected, dried and crystallized from a mixture of methanol/methylene chloride to obtain the title compound (0.68 g; m.p. 230 ° C. (d)).

EXAMPLE 5

3-(Oxazolo[4,5-b]pyridine-2-ylmethyl)-4-oxo-3H-phthalazin-1-ylacetc acid (I; X = O; $R_1$ = OH; $R_3$ = $R_4$ = H; $R_2$ = oxazolo[4,5-b]pyridine-2-yl)

A solution obtained by adding ethyl 4-oxo-3H-phthalazin-1-ylacetate (1.25 g) to a suspension of sodium hydride (285 mg; 50% w/w dispersion in mineral oil) in dimethylformamide (10 ml) was added dropwise to a solution of 2-chloromethyl-oxazolo[4,5-b]pyridine (1.0 g) in dimethylformamide (5 ml). After 2 hours, the reaction mixture was poured onto cold water (20 ml) and extracted with ether. The ether extract was washed with water (2 × 50 ml), dried and evaporated. The resulting crude material was chromatographed over silica gel to obtain 3-(oxazolo[4,5-b] pyridine-2-ylmethyl)-4-oxo-3H-phthalazin-1-ylacetate (m.p. 105-107 ° C) which was used directly in the next step. The product was dissolved in methanol (5 ml) containing 20% aqueous potassium hydroxide (0.5 ml) and heated on a steambath for 15 minutes. The solution was evaporated to dryness, the residue dissolved in water and acidified with acetic acid (2 ml). The resulting yellow precipitate was collected, triturated with hot methanol and filtered to obtain the title compound as a white solid (0.32 g; m.p. 228-230 ° C).

EXAMPLE 6

A solution of methyl 3-(2-benzothiazolyl)-4-oxo-3H-phthalazin-1-ylacetate (1.92 g) in methanol (50 ml) containing 10% aqueous potassium hydroxide (5 ml) was stirred at room temperature for 4 hours. The solution was concentrated to remove methanol and the concentrate was diluted with water (75 ml) and then extracted with ethyl acetate. The aqueous portion was separated and acidified with concentrated hydrochloric acid to pH 2.0. The precipitated solid was collected and crystallized from isopropyl alcohol to give 3-(2-benzothiazolyl)-4-oxo-3-H-phthalazin-1-ylacetic acid (876 mg; m.p. 205° C(d)).

EXAMPLE 7

In accordance with Example 6, the following compounds are prepared:

TABLE 1A

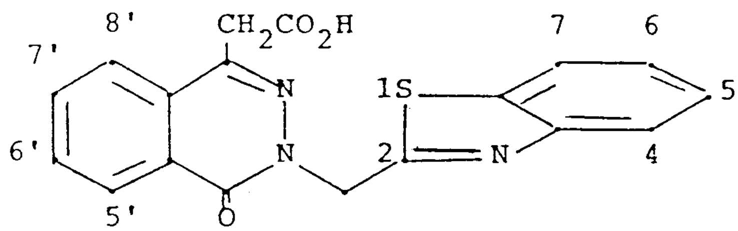

| Substituent | M.P.°C |
|---|---|
| H | 205(d) |
| 4-Cl | 217(d) |
| 5-Cl | 210-212 |
| 6-Cl | 207(d) |
| 5-Br | 214 |
| 6-Br | 214 |
| 7-Br | 173-175 |
| 5-$SCH_3$ | 187-188 |
| 5-$SOCH_3$ | 184(d) |
| 5-$SO_2CH_3$ | 210-211(d) |
| 4-Cl,5-Cl | 222 |
| 5-Cl,6-Cl | 192-195 |
| 4-Cl,6-Cl | 188-190 |
| 4-Cl,7-Cl | 223-224 |
| 5-Cl,7-Cl | 213 |
| 5-$CH_3$ | 205(d) |
| 6-$CH_3$ | 202(d) |
| 6-$OCH_3$ | 189(d) |
| 5'-F | 204-205 |
| 5'-$CH_3$ | 201-203 |
| 6,7-Benzo,5'-F | 218-222 |
| 6,7-Benzo,5'-$CH_3$ | 215-219 |
| 6'-Cl | 198-199 |
| 7'-Cl | 199 |
| 6'-Cl,7'-Cl | 189-192 |
| 6-Br,6'-Cl,7'Cl | 206(d) |
| 6'-Br | 211 |
| 6'-$CF_3$ | 210-211 |
| 6'-$NO_2$ | 199-201 |
| 6'-$OCH_3$ | 177-179 |

| Substituent | M.P.°C |
|---|---|
| 7'-Br | 192 |
| 7'-$CH_3$ | 187-190 |
| 7'-$OCH_3$ | 198-202 |
| 7'-$CF_3$ | 124-126 |
| 7'-$NO_2$ | 155-158 |
| 6,7-Benzo,7'-Cl | 209-210 |
| 5-$CF_3$ | 197-198 |
| 6'-isopropyl | 184-185 |
| 7'-isopropyl | 99-101 |
| 4-F | 217-218 |
| 4-F,5-F | 178-181 |
| 5-F | 222(d) |
| 6-isopropyl | 160-161 |

TABLE 1B

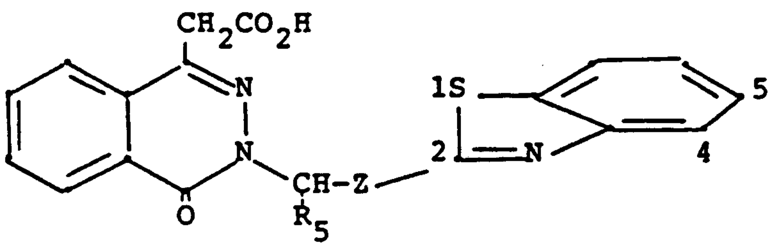

| Z | $R_5$ | Substituent | M.P.°C |
|---|---|---|---|
| covalent bond | $CH_3$ | - | 159-160(d) |
| covalent bond | $CH_3$ | 5-$F_3$ | 182-183 |
| covalent bond | $CH_3$ | 5-Cl | 205 |
| sulphur | H | - | 150-160 |
| sulphur | H | 5-Cl | 100-104 |

EXAMPLE 8

A mixture of methyl 4-oxo-3H-phthazalin-1-ylacetate (1.09 g) and sodium hydride (0.269 g; 50% w/w dispersion in mineral oil) in dimethylformamide (25 ml) was stirred at room temperature for two hours under nitrogen. To the solution obtained was added 2-bromomethylbenzothiazole (1.14 g) dissolved in dimethylformamide (5 ml), the reaction mixture was stirred for an additional one hour and then poured into ice water (50 ml). The pH of the mixture was brought to 4.0 by addition of 10% hydrochloric acid (5 ml) and extracted with ethyl acetate (100 ml). The extract was washed with water (50 ml), dried ($MgSO_4$) and evaporated. The crude product (1.92 g), methyl (2-benzothiazolyl)-4-oxo-3H-phthalazin-1-ylacetate, was characterized by NMR spectrum (see Table 2). The other compounds of Table 2 were prepared in a similar manner using the appropriate 4-oxo-3H-phthalazin-1-ylacetate and 2-bromomethylbenzothiazoles. The melting points are in degrees Centigrade.

TABLE 2A

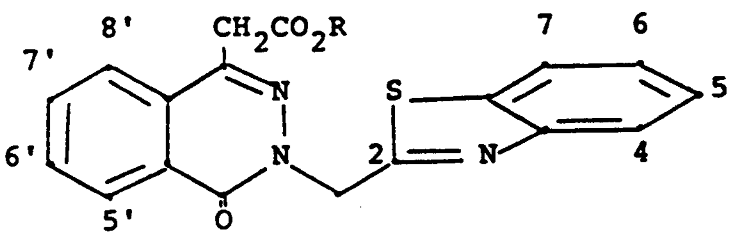

| Substituent | R | Product |
|---|---|---|
| H | $CH_3$ | 'HNMR($CDCl_3$,60MHz) 3.6(s,3H) 4.0(s,2H), 5.7(s,2H), 7.2(m,2H), 7.6(m,5H), 8.4(m,1H) |
| 4-F | $C_2H_5$ | m.p. 119-120 |
| 5-F | $C_2H_5$ | m.p. 118-120 |
| 4-Cl | $C_2H_5$ | m.p. 113-116 |

TABLE 2A (Continued)

| Substituent | R | Product |
|---|---|---|
| 5-Cl | $C_2H_5$ | m.p. 152-155 |
| 5-Br | $CH_3$ | m.p. 160-161 |
| 7-Br | $C_2H_5$ | 'HNMR($CDCl_3$,60MHz):1.2 (t,J=8Hz,3H),4.1(s,2H),4.2 (q,J=8Hz,2H),5.8(s,2H),7.3 (m,2H),7.8(m,4H),8.4(m,1H) |
| 5-$CH_3$ | $C_2H_5$ | m.p. 134-136 |
| 4-F,5-F | $C_2H_5$ | m.p. 118-122 |
| 4-Cl,5-Cl | $C_2H_5$ | m.p.121-122 |
| 5-Cl,6-Cl | $CH_3$ | 'HNMR($CDCl_3$,90MHz):3.70 (s,3H),4.00(s,2H),5.75(s,2H), 7.6-8.0(m,5H),8.3-8.6(m,1H) |
| 5-Cl,7-Cl | $C_2H_5$ | m.p. 144-145 |
| 4-Cl,6-Cl | $CH_3$ | 'HNMR($CDCl_3$,90MHz):1.20(t,J= 8Hz,3H),4.00(s,2H),4.15(q, J=8Hz,2H),5.80(s,2H)7.40 (d,J=1Hz,1H),7.60(d,J=1Hz,1H), 7.6-7.9(m,3H),8.4-8.6(m,1H) |
| 4-Cl,7-Cl | $C_2H_5$ | m.p. 173 |
| 6-$OCH_3$ | $CH_3$ | 'HNMR($CDCl_3$,90MHz):3.70 (s,3H),3.80(s,3H),4.05(s,2H), 5.75(s,2H),7.00(dd,J=3,9Hz,1H) 7.20(d,J=3Hz,1H),7.6-7.9,(m, 4H),8.4-8.6(m,1H) |
| 5-$CH_3$ | $C_2H_5$ | m.p. 123-124 |
| 6-$CH_3$ | $CH_3$ | 'HNMR($CDCl_3$,90MHz):2.50 (s,3H),3.75(s,3H),4.10(s,2H), 5.90(s,2H),7.35(d,J=8Hz,1H), 7.65-8.10(m,4H),8.5-8.7(m,1H) |

TABLE 2A (Continued)

| Substituent | R | Product |
| --- | --- | --- |
| 6-isopropyl | $C_2H_5$ | H'NMR($CDCl_3$,90MHz):8.40 (m,1H),7.9-7.5(m,5H),7.2(d,J=9Hz,1H),5.79(s,2H),4.15(q,J=9Hz,2H),3.85(s,2H),2.98(Sep.,J=9Hz,1H),1.28(d,J=9Hz,6H),1.20(5,J=9Hz,3H) |
| 5'-F | $C_2H_5$ | 'HNMR($CDCl_3$,90MHz):1.20(t,J=8Hz,3H),4.00(s,2H),4.20(q,J=8Hz,2H),5.80(s,2H),7.2-8.1 (m,7H) |
| 5'-$CH_3$ | $C_2H_5$ | 'HNMR($CDCl_3$,90MHz):1.20(t,J=8Hz,3H),2.95(s,3H),3.95 (s,2H),4.20(q,J=8Hz,2H),5.75(s,2H),7.2-8.1(m,7H) |
| 6,7-Benzo,5'-F | $C_2H_5$ | 'HNMR($CDCl_3$,90MHz):1.20(t,J=8Hz,3H),3.95(s,2H),4.20(q,J=8Hz,2H),5.90(s,2H),7.25-8.00(m,8H),8.80(dd,J=3,7Hz,1H) |
| 6,7-Benzo,5'-$CH_3$ | $C_2H_5$ | 'HNMR($CDCl_3$,90MHz):1.20(t,J=8Hz,3H),2.95(s,3H),3.95 (s,2H),4.15(q,J=8Hz,2H),5.90 (s,2H),7.4-7.95(m,8H),8.80 (m,1H) |
| 6'-Cl | $C_2H_5$ | 'HNMR($CDCl_3$,90MHz):1.20(t,J=8Hz,3H),4.05(s,2H),4.25 (q,J=8Hz,2H),5.85(s,2H),7.2-8.1(m,6H),8.50(d,J=2Hz,1H) |
| 7'-Cl | $C_2H_5$ | 'HNMR($CDCl_3$,90MHz):1.25(t,J=8Hz,3H),4.00(s,2H),4.20 (q,J=8Hz,2H),5.80(s,2H),7.25-7.6(m,2H),7.65-7.90 (m,3H),8.00(dd,J=2,9Hz,1H),8.45(d,J=9Hz,1H) |

TABLE 2A (Continued)

| Substituent | R | Product |
|---|---|---|
| 6'-Cl,7'Cl | $C_2H_5$ | 'HNMR($CDCl_3$,90MHz):1.20(t, J=8Hz,3H),4.00(s,2H),4.20(q, J=8Hz,2H),5.80(s,2H),7.2-7.6 (m,2H),7.7-8.1(m,3H),8.55 (s,1H) |
| 6'-Br | $C_2H_5$ | 'HNMR($CDCl_3$,90MHz):1.20(t, J=8Hz,3H),3.90(s,2H),4.15(q, J=8Hz,2H),5.80(s,2H),7.2-8.1 (m,6H),8.60(d,J=2Hz,1H) |
| 6'-$NO_2$ | $C_2H_5$ | 'HNMR($CDCl_3$,90MHz):1.20(t, J=8Hz,3H),4.05(s,2H),4.20(q, J=8Hz,2H),5.80(s,2H),7.2-7.6 (m,2H),7.7-8.1(m,3H),8.60(dd, J=2,9Hz,1H),9.30(d,J=3Hz,1H) |
| 6'-isopropyl | $C_2H_5$ | 'HNMR($CDCl_3$,90MHz):8.25(s, 1H),7.9(m,1H),7.7(m,3H),7.3 (m,2H),5.8(s,2H),4.20(q,J=9Hz, 2H),3.95(s,2H),3.1(sep.,J=9Hz, 1H),1.35(d,J=9Hz,6H),1.20(s, J=9Hz,3H) |
| 6'-$OCH_3$ | $C_2H_5$ | 'HNMR($CDCl_3$,90MHz):1.20(t, J=8Hz,3H),3.95(brs,6H),4.15(q, J=8Hz,2H),5.80(s,2H),7.20-8.10 (m,7H) |
| 6'-$CF_3$ | $C_2H_5$ | 'HNMR(90MHz,$CDCl_3$):8.7(s,1H), 8.15-7.7(m,4H),7.40(m,2H),5.8 (s,2H),4.21(q,J=9Hz,2H),4.0 (s,2H),1.22(5,J=9Hz,3H) |
| 7'Br | $C_2H_5$ | 'HNMR($CDCl_3$,90MHz):1.20(t, J=8Hz,3H),3.90(s,2H),4.15(q, J=8Hz,2H),5.85(s,2H),7.15-7.50 (m,2H),7.6-8.1(m,4H),8.30 (d,J=9Hz) |
| 7'-$CH_3$ | $C_2H_5$ | 'HNMR($CDCl_3$,90MHz):1.20(t, J=8Hz,3H),2.60(s,3H),4.15 (s,2H),4.20(q,J=8Hz,2H),5.80 (s,2H),7.3-8.2(m,6H),8.30(d, J=9Hz,1H) |

TABLE 2A (Continued)

| Substituent | R | Product |
|---|---|---|
| 7'isopropyl | $C_2H_5$ | 'HNMR(300MHz,$CDCl_3$):8.24 (d,J=9Hz,1H),7.84(d,J=9Hz, 1H),7.62(d,J=9Hz,1H),7.49 (d,J=9Hz,1H),7.36(s,1H), 7.26("t",J=6Hz,1H),7.16 ("t",J=6Hz,1H),5.64(s,2H), 4.06(q,J=9Hz,1H),1.30(d, J=9Hz,6H),1.17(t,J=9Hz,3H) |
| 7'$OCH_3$ | $C_2H_5$ | 'HNMR($CDCl_3$,90MHz):1.20(t, J=8Hz,3H),3.85(s,3H),3.90 (s,2H),4.20(q,J=8Hz,2H), 5.80(s,2H),7.00(d,J=2Hz,1H) 7.2-7.5(m,3H),7.75(dd,J=2.8Hz, 1H),8.00(dd,J=2.7Hz,1H),8.50 (d,J=9Hz,1H) |
| 7'$CF_3$ | $C_2H_5$ | 'HNMR(90MHz,$CDCl_3$):8.90(d, J=9Hz,1H),8.15-7.70(m,4H), 7.45-7.2(m,2H),5.8(s,2H), 4.20(q,J=9Hz,2H),4.05(s, 2H),1.18(t,J=9Hz,3H) |
| 7'-$NO_2$ | $C_2H_5$ | 'HNMR($CDCl_3$,90MHz):1.20(t, J=8Hz,3H),4.05(s,2H),4.20 (q,J=8Hz,3H),5.80(s,2H), 7.2-7.6(m,3H),7.6-8.1(m, 2H),8.4-8.7(m,2H) |
| 6,7-Benzo,7'-Cl | $C_2H_5$ | 'HNMR($CDCl_3$,90MHz):1.20(t, J=8Hz,3H),4.00(s,2H),4.20(q, J=8Hz,2H),5.90(s,2H),7.5- 8.0(m,7H),8.45(d,J=9Hz,1H), 8.75-8.90(m,1H) |
| 5-$CF_3$ | $C_2H_5$ | m.p. 134-136°C |

TABLE 2B

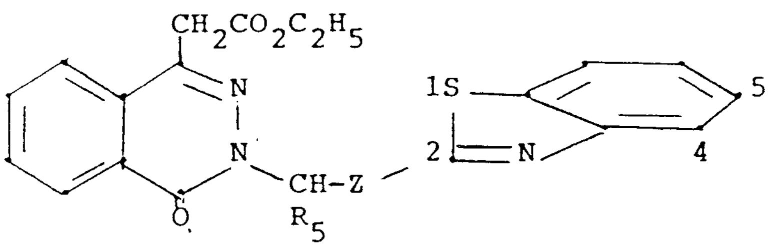

| Z | $R_5$ | Substituent | Product |
|---|---|---|---|
| covalent bond | $CH_3$ | - | m.p. 117-118°C |
| covalent bond | $CH_3$ | 5-$CF_3$ | m.p. 105-106°C |
| covalent bond | $CH_3$ | 5-Cl | m.p. 86-88°C |
| S | H | - | 'HNMR($CDCl_3$,60MHz): 1.2(t,J=9Hz,3H),3.85 (s,2H),4.2(9,J=9Hz, 2H),6.0(s,2H),7.1-8.0(m,7H),8.2-8.4 (m,1H) |

EXAMPLE 9

To a mixture of ethyl-4-oxo-3H-phthalazin-1-ylacetate (23.4g) in dimethylformamide (175 ml) maintained at 10°C was added potassium t-butoxide (11.2g) portion-wise over 5 minutes. The resulting orange-colored solution was brought to room temperature and 5-trifluoromethyl-2-chloromethylbenzothiazole in dimethylformamide (25 ml) was gradually added over 15 minutes. After stirring for an additional 30 minutes, the mixture was poured onto ice water (1500 ml). The precipitated solid was collected and washed with a mixture of isopropyl ether/n-hexane (250 ml/500 ml). The resulting light yellow solid, ethyl-3-(5-trifluoromethyl-2-benzothiazolylmethyl)-4-oxo-phthalazin-lylacetate, was air dried (44.3g), m.p. 134-136°C.

EXAMPLE 10

According to the method of Example 1D, the following compounds were prepared:

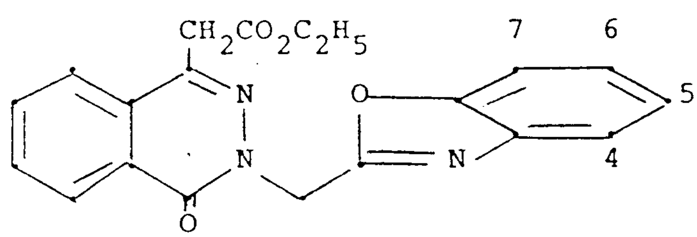

| Benzothiazolyl substituent | Product |
|---|---|
| H | not isolated |
| 5-Cl | not isolated |
| 5-Br | not isolated |
| 5-Cl,7-Cl | Mass Spectrum, m/e base peak 431.06 (partly isolated) |
| 5,6-benzo | m.p. 167-170 ° C |

According to the method of Example 6, the following compounds were prepared from the above compounds without isolation thereof.

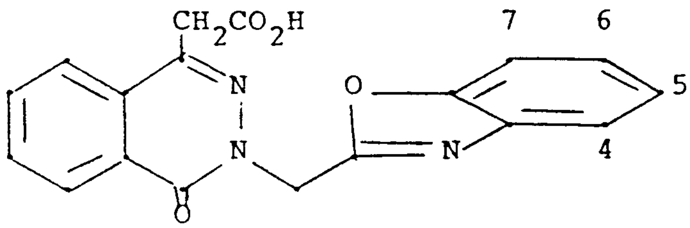

| Benzothiazolyl substituent | M.P. ° C |
|---|---|
| H | 179-183 |
| 5-Cl | 205-207 |
| 5-Br | 190-192 |
| 5-Cl,7-Cl | 199-201 |
| 5,6-benzo | 167-170 |

Example 11

In accordance with Example 3B, the following compounds were prepared.

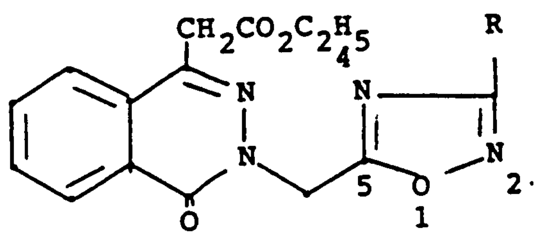

| R | Product |
|---|---|
| 2-F,6-Cl-phenyl | 'HNMR(CDCl3,60MHz): 1.3(t,J = 8Hz,3H),4.0 (s,2H),4.2(q,J = 8Hz,2H) 5.8(s,2H),7.3(m,3H), 7.8(m,3H),8.4(m,1H) |
| 2-pyridyl | m.p. 118-123 ° C |
| 2-Br-phenyl | 'HNMR(CDCl3,60MHz): 1.3(t,J = 8Hz,3H),4.0 (s,2H),4.2(q,J = 8Hz, 2H),5.7(s,2H),7.2 (m,2H),7.8(m,4H),8.4 (m,1H) |
| benzyl | m.p. 76-80 ° C |
| 2-F-phenyl | m.p. 100-105 ° C |
| 3-Cl,4-Cl-phenyl | m.p. 138-140 ° C |

EXAMPLE 12

In accordance with Example 3C, the following compounds were prepared.

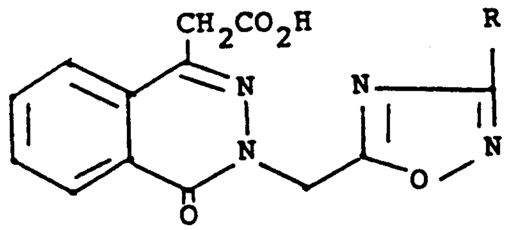

| R | M.P. ° C |
|---|---|
| 2-Cl-phenyl | 164-167 |
| phenyl | 202-205 |
| 4-Br phenyl | 193-195 |
| 2-methylphenyl | 182-184 |
| 2-$OCH_3$-phenyl | 174-175 |
| 2-F,6-Cl-phenyl | 178-182 |
| 3-Cl,4-Cl-phenyl | 220-221 |
| 2-pyridyl | 196-200 |
| 2-Br phenyl | 171-173 |
| benzyl | 56-60 |
| 2-F phenyl | 210-211 |

EXAMPLE 13

3-(Quinolin-2-ylmethyl)-4-oxo-3H-6,7-dichlorophthalazin-lylacetic acid. (I; X = O; $R_1$ = OH; $R_2$ = quinolin-2-yl; $R_3$ = $R_4$ = Cl)

A. 4,5-Dichlorophthalic Anhydride

A mixture of commercially available 4,5-dichlorophthalic acid (50.4 g) and acetic anhydride (150 ml) was refluxed for 2 hours. Upon cooling the precipitate product was collected and dried in vacuum (37.0 g; m.p. 180-181 ° C).

B. 3-Ethoxycarbonylmethylidene-5,6-dichlorophthalide

A solution of 4,5-dichlorophthalic anhydride (10.0 g) and (carbethoxymethylene)triphenylphosphorane (16.0 g) in chloroform (450 ml) was refluxed for 16 hours. Evaporation of chloroform and chromatography of the residue over silica gel gave the product (9.34 g).

C. Ethyl 6,7-dichloro-4-oxo-3H-phthalazin-1-ylacetate

A mixture of 3-ethoxycarbonylmethylidene-5,6-dichlorophthalide (9.37 g), ethanol (300 ml) and hydrazine (1.1 ml) was refluxed for 3 hours. Upon cooling, the precipitated solid was collected (6.85 g.; mass spectrum, m/e 300 and 227).

D. The title compound was prepared from ethyl 6,7-dichloro-4-oxo-3H-phthalazin-1-ylacetate and 2-chloromethylquinoline in accordance with the procedure in Example 6, m.p. 202-203 ° C.

EXAMPLE 14

3-(Quinolin-2-ylmethyl)-4-oxo-3H-phthalazin-1-ylacetic acid (I; X = 0; $R_1$ = OH; $R_3$ = $R_4$ = H; $R_2$ = quinolin-2-yl).

To a solution of ethyl-4-oxo-3H-phthalazin-1ylacetate (1.0 g) and sodium hydride (60% w/w dispersion in mineral oil) in dimethylformamide (30 ml) was added 2-bromomethylquinoline (1.05 g). The resulting

solution was stirred at room temperature for 30 minutes, poured onto water (100 ml) containing 1N HCl (5 ml), and extracted with ethyl acetate. The organic extract was washed with water (3×50 ml), dried and evaporated to obtain ethyl 3-(quinolin-2ylmethyl)-4-oxo-phthalazin-1-ylacetate (1.54 g). This substance was dissolved in a water/dioxane mixture (70 ml/70 ml). To the solution was added 5N potassium hydroxide (5 ml). The solution was allowed to stir at room temperature for 15 minutes, concentrated to remove excess dioxane, diluted with water (150 ml) and extracted with ethyl acetate (3×70 ml). The aqueous layer was adjusted to pH 2 with concentrated HCl. The precipitated solid was triturated with hot ethyl acetate and then filtered to obtain the title compound (0.54 g; m.p. 193-194 °C).

EXAMPLE 15

In accordance with Example 6, the following compounds were prepared.

**TABLE 3**

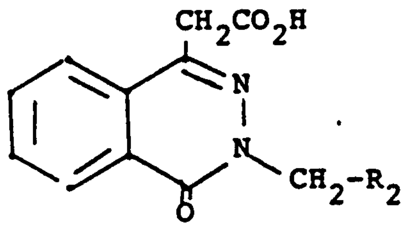

.

| $R_2$ | M.P. °C |
|---|---|
| benzisothiazol-3-yl | 168 |
| 5-(2-chlorophenyl)-1,2,4-oxadiazol-3-yl | 163-165 |
| 3-phenylisothiazol-5-yl | 169-170 |
| 3-phenylisothiazol-4-yl | 218 |
| 2-phenyl-1,3,4-oxadiazol-5-yl | 260 |
| 4,5-diphenyloxazol-2-yl | 197-200 |
| quinolin-2-yl | 193-194 |
| quinoxalin-2-yl | 215-217 |
| N-methylbenzimidazol-2-yl | 230(d) |
| oxazolo[4,5-b]pyridine-2-yl | 228-230 |
| thiazolo[5,4-b]pyridine-2-yl | 216 |
| 5,7-dichloroquinolin-2-yl | 200-201 |
| 6-bromoquinolin-2-yl | 205-206 |
| 6,8-dichloroquinolin-2-yl | 193-195 |
| 3-methyl-1,2,5-thiadiazol-4-yl | 160-162 |
| 5-phenyloxazol-2-yl | 159-162 |
| 4-phenyloxazol-2-yl | 181-184 |
| 2-phenylthiazol-5-yl | 164-165 |
| 2-O-fluorophenylthiazol-5-yl | 184-185 |
| 4-phenylthiazol-2-yl | 181-184 |
| 5-chlorobenzothiophen-2-yl | 205-206 |

EXAMPLE 16

According to Example 8, the following compounds were prepared:

TABLE 4

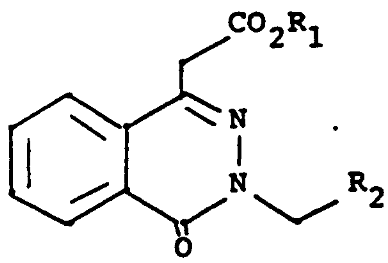

| $R_2$ | $R_1$ | Product |
|---|---|---|
| benzisothiazol-3-yl | $CH_3$ | 'HNMR($CDCl_3$,60MHz): 3.7(s,3H),4.0(s,2H), 5.8(s,2H),7.6(m,7H), 8.3(m,1H) |
| 3-phenylisothiazol-5-yl | $C_2H_5$ | m.p. 98-104°C |
| 3-phenylisothiazol-4-yl | $C_2H_5$ | 'HNMR($CDCl_3$,60MHz): 1.2(t,J=8Hz,3H),3.9 (s,2H),4.2(q,J=8Hz, 2H),5.4(s,2H)7.6 (m,7H),8.4(m,1H),8.8 (s,1H) |
| 2-phenyl-1,3,4-oxadiazol-5-yl | $C_2H_5$ | 'HNMR($CDCl_3$,60MHz): 1.2(t,J=8Hz,3H),4.0 (s,2H),4.2(q,J=8Hz, 2H),5.7(s,2H),7.4 (m,3H),7.8(m,5H),8.4 (m,1H) |
| 5-(2-chlorophenyl)-1,2,4-oxadiazol-3-yl | $C_2H_5$ | 'HNMR($CDCl_3$,60MHz): 1.3(t,J=8Hz,3H),4.0 (s,2H),4.2(q,J=8Hz, 2H),5.6(s,2H),7.4 (m,3H),7.8(m,4H),8.4 (m,1H) |
| N-methylbenzimidazol-2-yl | $C_2H_5$ | m.p. 118°C |

| $R_2$ | $R_1$ | Product |
|---|---|---|
| oxazolo[4,5-b]-pyridine-2-yl | $C_2H_5$ | m.p. 105-107°C |
| thiazolo[5,4-b]-pyridine-2-yl | $C_2H_5$ | m.p. 111-113°C |
| 5-phenyloxazol-2-yl | $C_2H_5$ | m.p. 115-117°C |
| 4-phenyloxazol-2-yl | $C_2H_5$ | m.p. 130-131°C |
| 2-phenylthiazol-5-yl | $C_2H_5$ | m.p. 104-106°C |
| 2-o-fluorophenyl thiazol-5-yl | $C_2H_5$ | m.p. 104-107°C |
| 4-phenylthiazol-2-yl | $C_2H_5$ | m.p. 120-124°C |
| 5-chlorobenzothio-phen-2-yl | $CH_3$ | m.p. 139-142°C |

EXAMPLE 17

(N-Morpholino)ethyl-3-(5,6-dichlorobenzothiazol-2-ylmethyl)-4-oxo-H-phthalazin-1-ylacetate, hydrochloride

To the sodium salt of N-2-hydroxyethylmorpholine, prepared by cautiously adding sodium hydride (0.45 g; 50% w/w dispersion in mineral oil) to a solution of N-2-hydroxyethylmorpholine(1.43 ml) in toluene (50 ml), was added a solution of ethyl 3-(benzothiazol-2-ylmethyl)-4-oxo-5,6-dichloro-phthalazin-1-ylacetate (1.23 g) in toluene (30 ml). After stirring the reaction mixture at room temperature for 24 hours and then at 60°C for 6 hours, it was exposed to HCl gas and the precipitated solid was added to saturated aqueous sodium bicarbonate (100 ml) and extracted with ethyl acetate (3 × 100 ml). The organic layer was dried and evaporated and the resulting solid was dissolved in acetone (30 ml). Exposure of this solution to gaseous HCl gave the title compound (0.12 g), which was characterized by elemental analysis (C, 49.83%; H,3.83%; N,9.36%).

EXAMPLE 18

Sodium 3-(5-trifluoromethylbenzothiazol-2-ylmethyl)-4-oxo-3H-phthalazin-1-ylacetate

Sodium methoxide (54 mg) was added to 3-(5-trifluoromethylbenzothiazol-2-ylmethyl)-4-oxo-phthalazin-1-ylacetic acid (0.4 g) in methanol (10 ml) at room temperature. After the addition was complete, a clear solution was obtained which was stirred for 15 minutes at room temperature. The excess methanol was evaporated. The residue was triturated with ether (20 ml) and filtered to obtain the pro t (0.43 g; m.p.> 300°C).

EXAMPLE 19

3-(5-Trifluoromethylbenzothiazol-2-ylmethyl)-4-oxo-3H-phthalazin-1 ylacetate, dicyclohexylamine salt

To a mixture of 3-(5-trifluromethylbenzothiazol-2ylmethyl)-4-oxo-phthalazin-1-ylacetic acid (0.42 g) in methanol (10 ml) was added dicyclohexylamine (0.2 g) in methanol (5 ml). The resulting clear solution was stirred at room temperature for 15 minutes and then evaporated to dryness. Trituration of the residue with ether (30 ml) gave a white solid (0.38 g; m.p. 207°C).

EXAMPLE 20

3-(5-Trifluoromethylbenzothiazol-2ylmethyl)-4-oxo-3H-phthalazin-1-ylacetic acid, meglumine salt

A solution of 3-(5-trifluoromethylbenzothiazol-2-ylmethyl)-4-oxo-phthalazin-1-ylacetic acid (419 mg) and meglumine (196 mg) in methanol (50 ml) was stirred at room temperature for an hour and then evaporated to dryness. The residue was triturated with ether (25 ml), filtered and the collected solid was air dried (610 mg; m.p. 157 ° C).

EXAMPLE 21

3-(5-Bromobenzothiazol-2-ylmethyl)-4-oxo-phthalazin-1-ylacetic acid, meglumine salt

A solution of 3-(5-bromobenzothiazol-2-ylmethyl)-4-oxo-phthalazin-1-ylacetic acid (430 mg) and meglumine (196 mg) in methanol (50. ml) was stirred at room temperature for an hour and evaporated to dryness. The residue was triturated with ether (25 ml) and the solid collected by filtration (620 mg; m.p. 138-140 ° C).

EXAMPLE 22

Ethyl 3-(5-sulfinylmethylbenzothiazole-2-ylmethyl)-4-oxo-3H-phthalazin-1-ylacetate

To an ice-cold solution of ethyl-(5-thiomethylbenzothiazole-2-ylmethyl)-4-oxo-phthalazin-1-ylacetate (1.06g) in chloroform (10ml) was added meta-chloroperoxybenzoic acid (0.50g). The resulting solution was stirred at between 0-5 ° C for 1 hour. The chloroform solution was washed with 10% sodium bicarbonate solution (3×20ml) and the separated organic layer was dried over magnesium sulfate and evaporated to dryness. The residue was purified by chromatography over silica gel to obtain the title compound [0.81g; ′HNMR ($CDCl_3$, 60 MHz): 1.2 (t, J = 8Hz, 3H), 2.65 (s, 3H), 3.95 (s, 2H), 4.1 (q, J = 8Hz, 2H), 5.75 (s, 2H), 7.4-8.3 (m, 7H)].

EXAMPLE 23

Ethyl 3-(5-sulfonylmethylbenzothiazole-2-ylmethyl)-4-oxo-3H-phthalazin-1-ylacetate

A solution of ethyl-(5-thiomethylbenzothiazole-2-ylmethyl)-4-oxo-3H-phthalazin-1-ylacetate (1.06g) and meta-chloroperoxybenzoic acid (1.3g) in chloroform (50ml) was stirred at room temperature for 1 hour. This solution was washed with 10% sodium bicarbonate solution (3×20ml) and the organic extract was dried and evaporated to obtain a light yellow solid (0.85g; ′HNMR ($CDCl_3$, 60MHz): 1.2 (t, J = Hz, 3H), 3.1 (s, 3H), 4.0 (s, 2H), 4.15 (q, J = 8Hz, 2H), 5.85 (s, 2H), 7.4-8.3 (m, 7H).

EXAMPLE 24

Ethyl 3-(5-trifluoromethyl-2-α-methyl benzothiazolyl)-4-oxo-3H-phthalazin-1-yl-acetate (; $R_1 = OC_2H_5$; $R_3 = R_4 = H$; $R_5 = CH_3$; $R_2$ = 5-trifluoromethyl-2-benzothiazolyl; X = O)

A. 1-(2-Benzothiazolyl)ethyl chloride

To a solution of 1-(2-benzothiazolyl)ethanol (2.5 g) prepared according to J. Indian Chem. Soc., 566 (1974) in methylene chloride (50 ml) was added thionyl chloride (3.32 g) and the resulting solution stirred at room temperature for 1 hour. The solution was poured onto ice-water (100 ml) and the organic extract separated. This extract was washed with aqueous bicarbonate (10 ml of a 5% solution) and then with water (50 ml). The methylene chloride layer was dried over anhydrous magnesium sulfate and evaporated to a light yellow oil (2.08 g).

B. 1-(5-Trifluoromethyl-2-benzothiazolyl)ethanol mesylate

To an ice-cold sol on of 1-(5-trifluoromethyl-2-benzothiazolyl)ethanol (m.p. 93-94 ° C, 4.94 g) in dry pyridine was added methanesulfonyl chloride (4.58 g) and the resulting solution stirred at 0 ° C for 1 hour. It was poured onto water, extracted with ether and the ether layer washed with 10% hydrochloric acid (2×20 ml). The organic extract was dried, evaporated and the residue triturated with hexane to obtain the desired

compound as solid, m.p. 89°C (5.89 g).

A mixture of ethyl 4-oxo-3H-phthalazin-1-ylacetate (2.34 g) and sodium hydride (0.53 g; 50% w/w dispersion in mineral oil) in dimethylformamide (25 ml) was stirred at room temperature for 30 minutes under nitrogen. To the solution obtained was added 1-(5-trifluoromethyl-2-benzothiazolyl)ethanol mesylate (3.2 g) dissolved in dimethylformamide (5 ml), the reaction mixture stirred for an additional hour and poured onto ice-water (50 ml). The pH of the mixture was brought to 4.0 by addition of 10% hydrochloric acid (5 ml) and extracted with ethyl acetate (100 ml). The extract was washed with water (50 ml), dried over anhydrous magnesium sulfate and evaporated. The crude product was purified by chromatography over silica gel by eluting with ethylacetate-chloroform (5/95% mixture). The title compound (2.0 g) was obtained by evaporation of eluants, m.p. 105-106°C.

EXAMPLE 25

3-(Benzothiazole-5-ylmethylbenzothiazolyl)-4-oxo-phthalazin-1-yl acetic acid

A. 5-Bromomethylbenzothiazole was prepared according to Example 1C starting from 5-methylbenzothiazole made according to Grazz. Chim. Ital., 95, 499 (1965). The products showed the following NMR resonances - TMS (60 MHz, $CDCl_3$): 4.6 (s,2H), 7.4 (dd, 1H,J=10Hz, J=2Hz),7.85 (d, 1H,J=10Hz), 8.15-(d,J=2Hz), 9.0(s,1H).

B. Ethyl 3-(5-methylbenzothiazolyl)-4-oxo-3H-phthalazin-1-yl-acetate prepared according to Example 1D had the following NMR resonances - TMS, (60MHz, $CDCl_3$): 1.2(t,J=8Hz,3H),3.95(s,2H),4.2(q,J=8Hz,2H)-,5.6 (s,2H),7.2-8.2(m,7H),9.0(s,1H).

C. The title compound was prepared according to Example 1E, m.p. 203-204°C.

EXAMPLE 26

Ethyl-3-(5-fluorobenzothiazolyl-2-ylmethyl)-4-oxo-phthalazin-ylacetate

To a mixture of ethyl 4-oxo-3-H-phthalazin-1-ylacetate (2.34 g) in dimethylformamide (15 ml) was added sodium methoxide (0.51 g). To the clear yellow solution obtained upon stirring the mixture for 5 minutes was added a solution of 5-fluoro-2-chloromethylbenzothiazole made in accordance with Example 1C in dimethylformamide (5 ml). After stirring the resulting solution for 1 hour, it was poured onto ice-water (50 ml) and extracted with methylene chloride. Evaporation of the organic extract gave a light orange colored solid, which was crystallized from ethanol to obtain the title compound (3.84 g, m.p. 118-120°C).

EXAMPLE A

In Table 5, the first two intermediates were prepared by the method of U.S. Patent 4,251,528 and the remaining intermediates by the method of Example 13C.

**TABLE 5**

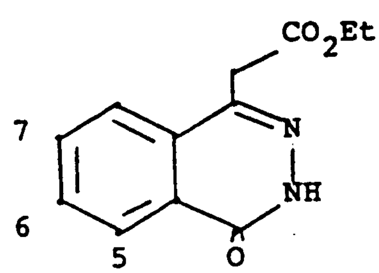

| Substituent | Product |
|---|---|
| 5-F | U.S. 4,251,528 |
| 5-$CH_3$ | U.S. 4,251,528 |
| 7-Cl | m.p. 189-190°C |
| 6-Cl,7-Cl | m.p. 250°C |
| 6-$NO_2$ | m.p. 224-225°C |
| 7-$CH_3$ | m.p. 228-230°C |
| 7-$OCH_3$ | m.p. 231-232°C |
| 7-$NO_2$ | m.p. 172-174°C |
| 6-isopropyl | TMS(300MHz,$CDCl_3$):8.28 (s,1H),7.63(d,J=3Hz,2H), 4.12(q,J=9Hz,2H),3.92(s, 2H),3.04(sep.,J=9Hz,1H), 1.25(d,J=9Hz,6H),1.16(t, J=6Hz,3H) |
| 7-isopropyl | TMS(300MHz,$CDCl_3$):8.32(d, J=9Hz,1H),7.65(dd,J=1Hz, 9Hz,1H),7.55(s,1H),4.15 (q,J=9Hz,2H),3.96(s,2H), 3.06(sep.,J=9Hz,1H),1.27 (d,J=9Hz,6H),1.18(t,J= 6Hz,3H) |
| 6-$CF_3$ | TMS(90MHz,DMSO-d6):13.0-12.6(br,1H),8.4(s,1H), 8.2-79(m,2H),4.00(q,J= 9Hz,2H),3.98(s,2H),1.0(t, J=9Hz,3H) |
| 7-$CF_3$ | TMS(90MHz,DMSO-d6):13.0-12.6(br,1H),8.5(d,J=9Hz, 1H),8.22(m,1H),8.1(s,1H), 4.10(1,J=9Hz,2H),4.10(s, 2H),1.15(t,J=9Hz,3H) |

EXAMPLE B

The following intermediates were prepared according to Example 1C unless otherwise indicated. All melting points are in degrees Centigrade.

## TABLE 6

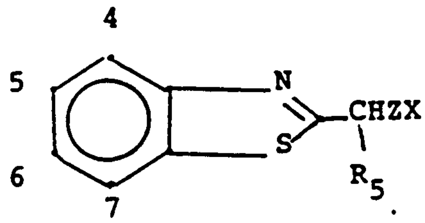

| Substituent | $R_5$ | Z | X | Product |
|---|---|---|---|---|
| 6-Cl | H | – | Br | 'HNMR($CDCl_3$,60MHz):4.75(s,2H), 7.35(dd,J=2,8Hz,1H),7.75(d,J=2.8Hz,1H),7.85(d,J=8Hz,1H) |
| 5-Br | H | – | Br | m.p. 107 |
| 6-Br | H | – | Br | m.p. 108 |
| 7-Br | H | – | Br | 'HNMR($CDCl_3$,60MHz):4.7(s,2H), 7.2-8.0(m,3H) |
| 5-Cl,6-Cl | H | – | Br | 'HNMR($CDCl_3$,90MHz):4.80(s,2H), 8.00(s,1H),8.15(s,1H) |
| 4-Cl,6-Cl | H | – | Br | m.p. 131-134°C<br>'HNMR($CDCl_3$,90MHz):4.80(s,2H), 7.45(d,J=1Hz),7.65(d,J=1Hz,1H) |
| 6-$OCH_3$ | H | – | Br | 'HNMR($CDCl_3$,90MHz):3.85(s,3H), 4.75(s,2H),7.05(dd,J=2,9Hz, 1H),7.25(d,J=2Hz,1H),7.85(d,J=9Hz,(1H) |
| 6-$CH_3$ | H | – | Br | 'HNMR($CDCl_3$,90MHz):2.45(s,3H) 4.90(s,2H),7.25(d,J=7Hz,1H), 7.60(s,1H),7.85(d,J=8Hz,1H) |
| 5-Cl | H | – | Cl | m.p. 78-80 |
| 4-Cl* | H | – | Cl | m.p. 114-115 |
| 4-F* | H | – | Cl | 'HNMR(60MHz,$CDCl_3$):4.9(s, 2H),7.0-79(m,3H) |
| 4-F,5-F* | H | – | Cl | m.p. 68-70 |
| 5-Cl,7-Cl* | H | – | Cl | m.p. 74-76 |

| Substituent | $R_5$ | Z | X | Product |
|---|---|---|---|---|
| 4-Cl,5-Cl | H | - | Cl | 'HNMR(60MHz,$CDCl_3$):4.7(s,2H), 6.6(d2H,J=10Hz),7.2(d,2H,J=10Hz) |
| 4-Cl,7-Cl | | | | m.p. 134-135 |
| 5-$CH_3$ | H | - | Cl | m.p. 114-116 |
| 6-isopropyl | H | - | Cl | 'HNMR(90MHz,$CDCl_3$):7.85(d,J=9Hz,1H),7.65(d,J=3Hz,1H),7.40(dd,J=3Hz,9Hz,1H),4.9(s,2H),3.00(sep.,J=6Hz,1H)1.35(d,J=6Hz,6H) |
| H | $CH_3$ | - | Cl | 'HNMR(60MHz,$CDCl_3$):2.0(d,3H,J=8Hz),5.4(q,1H,J=8Hz),7.0-8.0(m,4H) |
| 5-$CF_3$ | $CH_3$ | - | $OSO_2CH_3$ | m.p. 84 |
| 5-Cl | $CH_3$ | - | $OSO_2CH_3$ | m.p. 92-94 |
| H | H | S | Cl | 'HNMR(60MHz,$CDCl_3$):5.25(s,2H),7.0-8.0(m,4H) |
| 5-Cl | H | S | Cl | 'HNMR(60MHz,$CDCl_3$):5.25(s,2H),7.0-8.0(m,3H) |

* Prepared according to Can. J. Chem., 43, 2610 (1965)

EXAMPLE C

The following intermediates were prepared by the indicated methods of the prior art, or by the method of Example 1B.

TABLE 7

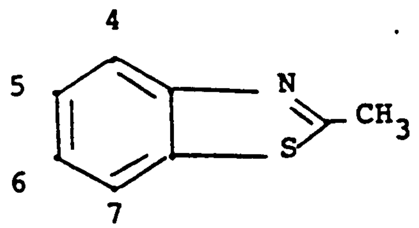

| Substituent | Product |
|---|---|
| 5-bromo | Chemical Abstracts; 1957 52,6319 |
| 6-bromo | J. Chem. Soc.; 1936, 1225 |
| 7-bromo | Liquid; m/e 228 |
| 5-Cl,6-Cl | m.p. 134-135°C |
| 6-$CH_3$ | Prepared similarly to procedures in J. Chem. Soc.; 1922, 1493 and J. Org. Chem.; 1976, 41,776 |
| 4Cl,6-Cl | Synthesis; 1976,730 |
| 6-Cl | Synthesis; 1976, 730 |

EXAMPLE D

5-(2-Chlorophenyl)-2-chloromethyl-1,2,4-oxadiazole

A. 0-2-Chlorobenzoyl-chloroacetamidoxime

Chloracetamidoxime (5 g) was dissolved in warm benzene (20 ml) and 2-chlorobenzoyl chloride (6.4 ml) was cautiously added to it. The resulting mixture was heated at 40° C for 30 minutes and excess benzene was removed. After cooling to room temperature the solidifed mass was triturated with cold benzene and filtered to obtain the compound 1.6 g; m.p. 126-130° C).

B. 0-2-chlorobenzoyl-chloroacetamidoxime (1.4 g) was added to refluxing diglyme (10 ml) and the refluxing continued for 10 minutes. After cooling, the reaction mixture was poured onto water (20 ml) containing ether (50 ml). The ether layer was washed with water (2 × 50 ml), saturated aqueous sodium bicarbonate (2 × 10 ml) and the organic layer dried and evaporated. The residue was chromatographed over silica gel to obtain the product, 0.8 g of a colorless oil, ´HNMR($CDCl_3$,60MHz): 4.7 (s,2H), 7.45 (m, 3H), 8.1 (m, 1H).

EXAMPLE E

The commercially available 2-amino-3-hydroxypyridine (5.0 g) and diglyme (30 ml) were heated at 125° C to obtain a solution. To this solution was added 2-chloro-1,1,1-triethoxyethane (9.9g) and the resulting mixture held at 125° for 1 hour. The solution was cooled to room temperature and then decanted to remove a black byproduct residue. The filtrate was diluted with water (50 ml) and the resulting yellow precipitated 2-chloromethyl-oxazolo[4,5-b]pyridine was collected (1.5 g). A small sample was crystallized from isopropanol (m.p. 115-118° C).

2-Chloromethyl-thiazolo[5,4-b]pyridine was prepared similarly by heating a mixture of 3-amino-2-mercaptopyridine (3.6 g), 2-chloro-1,1,1-triethoxyethane (6.5 g) and ethanol (60 ml) at 60 °C for 4 hours. The crude solid resulting from evaporation of ethanol was chromatographed over silica gel to obtain 2.94 g of the product, m.p. 71-73 °C.

Similarly, 2-chloromethyl-5-trifluoromethylbenzothiazole, m.p. 52 °C, was formed from 2-amino-4-trifluoromethylthiophenol hydrochloride and 2-chloro-1,1,1-triethoxyethane in ethanol.

EXAMPLE F

Intermediates of the formula Hal-$CH_2R_2$ were prepared by the methods listed below or by the method of Example E.

TABLE 8

| Structure | Product |
|---|---|
| 3-bromomethylbenzisothiazole | R.A. Gillham, Jr., Ph.D. thesis, California Institute of Technology, 1969 |
| 3-phenyl-5-chloromethylisothiazole | 'HNMR($CDCl_3$,60MHz): 4.8(s,2H),7.4(m,3H), 7.9(m,2H) |
| 2-chloromethyl-5-phenyloxazole | m.p. 64-66° C |
| 2-chloromethyl-4-phenyloxazole | m.p. 50-52° C |
| 5-chloromethyl-2-phenylthiazole | U.K. Patent 1,137,529 |
| 5-chloromethyl-2-0-fluorophenylthiazole | U.K. Patent 1,137,529 |
| 3-phenyl-4-chloromethylisothiazole | 'HNMR($CDCl_3$,60MHz): 4.5(s,2H),7.4(m,3H), 7.7(m,2H) |
| 2-phenyl-5-chloromethyl1,3,4-oxadiazole | Chem. Ber. 96, 1049 (1963) |
| 2-chloromethyl-5-chlorobenzothiophene | J. Chem. Soc. (C), 731 (1967) |
| 3-chloromethyl-5-(2-chlorophenyl)1,2,4-oxadiazole | Prepared similarly to procedure in J. Heterocyclic Chem. 16, 1469 (1979). 'HNMR ($CDCl_3$,60MHz):4.7 (s,2H),7.45(m,3H),8.1 (m,1H) |
| N-methyl-2-chloromethylbenzimidazole | JACS, 65, 1854, (1943), m.p. 95° C |
| 3-methyl-4-bromomethyl-1,2,5-thiadiazole | J. Heterocyclic Chem., 21, 1157 (1984), b.p. 30-35° C at 7 mm |

EXAMPLE G

The benzoate of 4-phenylthiazole-2-methanol (7.4 g), prepared according to J. Am. Chem. Soc., 53, 1470 (1931) was dissolved in anhydrous tetrahydrofuran (50 ml). To this solution was added lithium aluminum hydride (525 mg) and the mixture stirred for one hour at room temperature. After carefully quenching the excess lithium aluminium hydride with ethyl acetate, the mixture was poured onto ice-cold water (50 ml) and acidified to pH 3 using 10% HCl. The mixture was filtered and the filtrate was extracted with ether (3x50ml). The organic extract wad dried, evaporated and the crude product was purified by chromatography to obtain 4-phenylthiazole-2-methanol (1.8 g). This compound was converted to 4-phenylthiazole-2-methanol, methane sulfonate according to the procedure described in Example 24B. The melting point of the product was 80 ° C.

EXAMPLE H

The following intermediates were prepared by the method of Helvitica Chimica Acta 49, 412 (1966) except for the last two intermediates which were prepared by the method of J. Med. Chem. 4,351 (1961).

TABLE 9

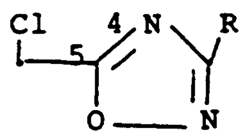

| R | Product |
|---|---|
| phenyl | Helvitica Chimica Acta 49, 412 (1966) |
| 2-Cl phenyl | 'HNMR($CDCl_3$,60MHz): 4.8(s,2H),7.4(m,3H), 7.9(m,1H) |
| 4-Br phenyl | m.p. 155-160°C |
| 2-methylphenyl | 'HNMR($CDCl_3$,60MHz): 2.6(s,3H),4.7(s,2H), 7.3(m,3H),8.0(m,1H) |
| 2-$OCH_3$ phenyl | m.p. 59-62°C |
| 2-F, 6-Cl phenyl | 'HNMR($CDCl_3$,60MHz): 4.8(s,2H),7.3(m,3H) |
| 2-F phenyl | m.p. 32-34°C |
| 3-Cl, 4-Cl phenyl | m.p. 38-41°C |
| 2-pyridyl | m.p. 89-94°C |
| 2-bromophenyl | m.p. 45-46°C |
| 2-$CF_3$ phenyl | 'HNMR($CDCl_3$,60MHz): 4.8(s,2H),7.8(m,4H) |
| benzyl | 'HNMR($CDCl_3$,60MHz): 4.0(s,2H),4.4(s,2H), 7.3(s,5H) |

EXAMPLE J

A mixture of 5-chloro-2-hydroxyaniline (10 g), chloroacetamidic acid ethyl esterhydrochloride (8.5 g) in chloroform (100 ml) was refluxed for 5 hours. The dark brown solution was filtered, washed first with 10% aqueous potassium hydroxide solution (10 ml) and then water (20 ml). The organic layer was dried (magnesium sulfate) and then evaporated to dryness. The residue was purified by column chromatography (m.p. 53-56 °C, yield, 8.6 g).

The following intermediates were prepared by the above procedure.

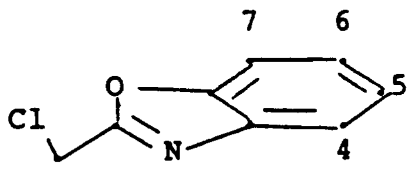

| Substituent | Product |
|---|---|
| 5-bromo | m.p. 63-65 °C |
| 5-Cl, 7-Cl | m.p. 52-53 °C |
| 5,6-dibenzo | m.p. 105 °C(d) |

Example K

A. A mixture of l,l,l-triethoxyethane (97.3 g) and N-chlorosuccinimide (88.l g) in carbon tetrachloride (600 ml) was warmed to 40 °C and then irradiated with an ultraviolet lamp. The reaction became exothermic and then subsided upon completion of the reaction. The precipitated succinimide byproduct was filtered off and the filtrate was concentrated to remove carbon tetrachloride. The residual liquid was distilled to obtain pure 2-chloro-l,l,l-triethoxyethane (9l.0 g; b.p. 9l °C/25 mm).

B. A solution of 2-chloro-l,l,l-triethoxyethane (5.9 g) and 2-aminothiophenol (2.5 g) was heated at 80 °C for l5 minutes. After cooling to room temperature, it was dissovled in methylene chloride (30 ml) and the resulting solution was washed with 3N HCl (l0 ml) and then with water (20 ml). The organic portion was evaporated and the residue chromatographed over silica gel to obtain 2-chloromethylbenzothiazole (3.35 g; 90% yield), m.p. 34 °C.

Example L

2-Chloromethyl-5-bromobenzothiazole

A mixture of crude 2-amino-4-bromothiophenol tin hydrochloride complex (7l.6 g) prepared according to JACS 53, 209 (l93l), 2-chloro-l,l,l-triethoxyethane (58.7 g) and ethanol (400 ml) was heated to gentle reflux for 30 minutes to obtain a solution. To the warm solution was added 3N HCl (l0 ml) and the precipitated solid was collected, washed with water and then dried to obtain the title compound (35.5 g), m.p. l07 °C.

Example M

A. 2-Amino-4-trifluromethylthiophenol, hydrochloride

The commercially available 4-chloro-3-nitrobenzotrifluoride (l00 g) was dissolved in ethanol (400 ml). To this was added portionwise a solution prepared by first adding sodium sulfide hydrate (80 g) to hot ethanol (200 ml) and then sulfur (9.6 g). After the intial exothermic reaction had subsided, the reaction mixture was refluxed for an additional 30 minutes and then cooled to room temperature. The precipiated yellow solid was collected, washed with cold ethanol and then dried to yield 4,4′-ditrifluoromethyl-2,2′-dinitrodiphenyldisulfide (63.0 g; m.p. l52-l54 °C). A mixture of this compound (62 g), tin metal (20 mesh, l32.0 g), ethanol (500 ml) and concentrated HCl (200 ml) was gently refluxed at 80 °C till a near solution was obtained. Then the reaction was maintained at 70 °C for 30 minutes. The warm solution was filtered and the filtrate concentrated under low pressure to a viscous liquid. To this was added 6N HCl and the precipitated white solid was filtered to obtain the title compound (49.0 g; m.p. 208-209 °C.).

B. 2-Chloromethyl-5-trifluoromethylbenzothiazole

To a solution of 2-amino-4-trifluoromethylthiophenol hydrochloride (30.0 g) in ethanol (l25 ml) was added 2-chloro-l,l,l-triethoxyethane (3l.0 g). The mixture was heated for l hour at 60 °C. The solution was concentrated to remove excess ethanol and the resulting material was extracted with ether (500 ml). The organic extract was washed successively with l0% HCl (20 ml), water (l00 ml), l0% sodium bicarbonate

solution and water (l00 ml), and then evaporated to obtain an amber colored oil, which crystallized upon standing at room temperature (28.9 g; m.p. 52 ° C).

Example N

2-Chloromethyl-5,7-dichlorobenzoxazole

To a solution of 2,4-dichloro-6-nitrophenol (l0.0 g) in water (450 ml) containing sodium bicarbonate (4.8 g) was added sodium dithionate in a quantity sufficient to turn the original dark solution colorless. The hot reaction mixture was filtered and the filtrate was cooled to room temperature and the crystallized product, 2-amino-4,6-dichlorophenol was collected (l.6 g). The 2-amino-4,6-dichlorophenol (l.6 g) was dissolved in ethanol (5 ml) and 2-chloro-l,l,l-triethoxyethane (l.9 g) was added. The resulting solution was warmed on a steambath for 1.5 hour. After cooling to room temperature, cold water (5 ml) was added. The precipitated solid was collected and then air dried to obtain the title compound (1.12 g; m.p. 52-53 ° C).

Example O

2-Chloromethyl-5-bromobenzoxazole

2-Amino-4-bromophenol (l.6 g) prepared according to U.S. Patent 4,157,444 was dissolved in ethanol (5 ml) and 2-chloro-l,l,l-triethoxyethane (l.9 g) was added. The resulting solution was warmed on a steambath for l.5 hour. After cooling the reaction mixture to room temperature, cold water (5 ml) was added. The precipitated solid was collected and air-dried to obtain the product (l.l2 g; m.p. 63-65 ° C.

Example P

By a method similar to that of Example K.B., 2, 2-chloromethyl-5-chlorobenzoxazole, m.p. 53-56 ° C., was prepared from commercially available 2-amino-4-chlorophenol using ethanol or chloroform as the solvent instead of methylene chloride. Similarly, 2-chloromethyl-5-methylthiobenzothiazole, m.p. 65-66 ° C and 2-chloromethyl-5-fluorobenzothiazole, m.p. 73 ° C, were prepared from 2-amino-4-methylthiophenol hydrochloride and 2-amino-4-fluorothiophenol hydrochloride, respectively, in ethanol solvent.

Example Q

5-Chloromethyl-2-phenyl-l,3,4-oxadiazole:

A mixture of 2-chloro-l,l,l-triethoxyethane (4.8 g), benzoyl hydrazine (3.0 g) and ethanol (30 ml) was refluxed for 2 hours and the solution was allowed to cool to room temperature. To the precipitated white crystalline solid was added water (l0 ml) containing a few drops of l0% HCl. The mixture was stirred for l0 minutes, and then filtered and the solid collected. The mother liquor was evaporated to dryness, the residue triturated with water and filtered to obtain a second crop of white solid. The combined yield of the two crops amounted to 3.8 g (89%; ′HNMR identical to the product described in Chem. Ber. 96, l049 (l969).

**Claims**

**Claims for the following Contracting States : BE, CH, DE, FR, GB, IT, LI, LU, NL, SE**

**1.** A compound of the formula

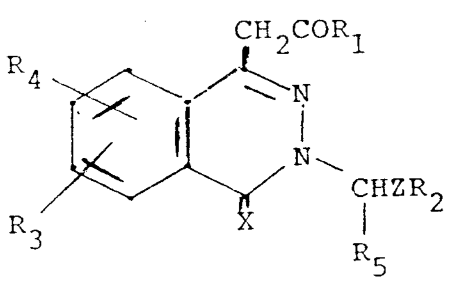

I

wherein

X is oxygen or sulfur;

Z is a covalent bond, O, S, NH or $CH_2$;

$R_1$ is hydroxy, or a conventional ester-forming residue which is convertible in vivo to hydroxy;

$R_2$ is a heterocyclic 5-membered ring having one nitrogen, oxygen or sulfur, two nitrogens one of which may be replaced by oxygen or sulfur, or three nitrogens one of which may be replaced by oxygen or sulfur, said ring substituted by one or two fluoro, chloro, ($C_1$-$C_4$)alkyl or phenyl, or condensed with benzo, or substituted by one of pyridyl, furyl or thienyl, said phenyl or benzo optionally substituted by one of iodo or trifluoromethylthio, or one or two of fluoro, chloro, bromo, ($C_1$-$C_4$)alkyl, ($C_1$-$C_4$)alkoxy, ($C_1$-$C_4$)-alkylthio, ($C_1$-$C_4$)alkylsulfinyl, ($C_1$-$C_4$)alkylsulfonyl, or trifluoromethyl, and said pyridyl, furyl or thienyl optionally substituted in the 3-position by fluoro, chloro, bromo, ($C_1$-$C_4$)alkyl or ($C_1$-$C_4$)alkoxy;

a heterocyclic 6-membered ring having one to three nitrogen atoms, or one or two nitrogen atoms and one oxygen or sulfur, and said ring substituted by one or two ($C_1$-$C_4$)alkyl or phenyl, or condensed with benzo, or substituted by one of pyridyl, furyl or thienyl, said phenyl or benzo optionally substituted by one of iodo or trifluoromethylthio, or one or two of fluoro, chloro, bromo, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl, or trifluoromethyl, and said pyridyl, furyl or thienyl optionally substituted in the 3-position by fluoro, chloro, ($C_1$-$C_4$)alkyl or ($C_1$-$C_4$)alkoxy;

oxazole or thiazole condensed with a 6-membered aromatic group containing one or two nitrogen atoms, with thiophene or with furane, each optionally substituted by one of fluoro, chloro, bromo, trifluoromethyl, methylthio or methylsulfinyl;

imidazolopyridine; naphthothiazole; or naphthoxazole;

$R_3$ and $R_4$ are the same or different and are hydrogen, fluoro, chloro, bromo, trifluoromethyl, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl, or nitro, or $R_3$ and $R_4$ taken together are ($C_1$-$C_4$)alkanedioxy; and $R_5$ is hydrogen or methyl; or

a pharmaceutically acceptable base addition salt of a compound of formula I wherein $R_1$ is hydroxy or an acid addition salt of a compound of formula I wherein $R_1$ is di($C_1$-$C_4$)alkylamino or ($C_1$-$C_4$)alkoxy substituted by N-morpholino or di($C_1$-$C_4$)alkylamino;

with the proviso that when $R_2$ is either a heterocyclic 5-membered ring having one heteroatom which is sulphur or a heterocyclic 6-membered ring having one heteroatom which is nitrogen, then said ring is either condensed with benzo, or substituted by one of pyridyl, furyl or thienyl, said benzo and said pyridyl, furyl and thienyl being optionally substituted as defined above.

**2.** A compound according to claim 1 characterized in that X is oxygen.

**3.** A compound according to claim 1 or 2 characterized in that $R^2$ is optionally substituted benzothiazolyl, benzoxazolyl, isoquinolyl, benzothiophen-yl, or benzofuran-yl, or substituted oxadiazolyl or indolyl.

**4.** A compound according to claim 1 characterized in that X is oxygen, Z is a covalent bond or $CH_2$, $R_1$ is hydroxy, $R_2$ is optionally substituted benzothiazol-2-yl, benzothiazol-5-yl, benzoisothiazol-3-yl, benzoxazol-2-yl, 2- quinolyl, 2-quinoxalyl, oxazolo[4,5-b]pyridine-2-yl, benzothiophen-2-yl, benzofuran-2-yl, or thiazolo[4,5-b]pyridine-2-yl, or substituted 1,2,4-oxadiazol-3-yl, 1,2,4-oxadiazol-5-yl, isothiazol-5-yl, isothiazol-4-yl, 1,3,4-oxadiazol-5-yl, 1,2,5-thiadiazol-3-yl, oxazol-2-yl, thiazol-2-yl, or thiazol-4-yl, and $R_3$, $R_4$ and $R_5$ are hydrogen.

**5.** A compound according to claim 1 or 2 characterized in that $R_2$ is 2-benzothiazolyl substituted in the benzo by one trifluoromethylthio, or one or two of chloro, bromo, methyl, methoxy, trifluoromethyl, or 6,7-benzo.

**6.** A compound according to claim 1, 2 or 3, characterized in that $R_3$ is hydrogen, 5-fluoro, 5-chloro, 5-bromo or 5-methyl, and $R_4$ is hydrogen; 6- or 7- substituted chloro, bromo, methyl, isopropyl, methoxy, nitro or trifluoromethyl; 4,5-difluoro, or 5,7-dichloro.

**7.** A compound according to claim 1 or 2, characterized in that $R_2$ is optionally substituted benzothiazol-2-yl or quinoxalyl, and $R_3$ and $R_4$ are each chloro.

**8.** A compound according to claim 1, characterized by being in the form of the sodium salt or the N-methylglucamine salt.

**9.** A pharmaceutical composition comprising a compound of any one of claims 1 to 8 in admixture with a pharmaceutically acceptable carrier.

**10.** A compound according to any one of claims 1 to 8 for use as a medicament.

**11.** The use of a compound of the formula (I) as claimed in any one of claims 1 to 8 for the manufacture of a medicament for use as an aldose reductase inhibitor.

**Claims for the following Contracting States : AT, ES, GR**

**1.** A process for the preparation of a compound of the formula

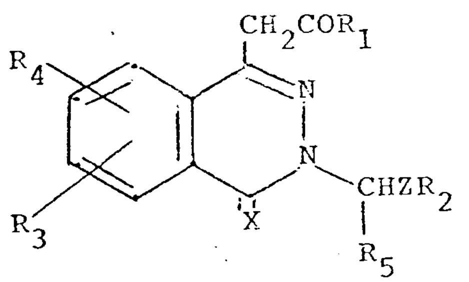

I

wherein

X is oxygen or sulfur;

Z is a covalent bond, O, S, NH or $CH_2$;

$R_1$ is hydroxy, or a conventional ester-forming residue which is convertible in vivo to hydroxy;

$R_2$ is a heterocyclic 5-membered ring having one nitrogen, oxygen or sulfur, two nitrogens one of which may be replaced by oxygen or sulfur, or three nitrogens one of which may be replaced by oxygen or sulfur, said ring substituted by one or two fluoro, chloro, ($C_1$-$C_4$) alkyl or phenyl, or condensed with benzo, or substituted by one of pyridyl, furyl or thienyl, said phenyl or benzo optionally substituted by one of iodo or trifluoromethylthio, or one or two of fluoro, choro, bromo, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl, or trifluoromethyl, and said pyridyl, furyl or thienyl optionally substituted in the 3-position by fluoro, chloro, bromo, ($C_1$-$C_4$)alkyl or ($C_1$-$C_4$)alkoxy;

a heterocyclic 6-membered ring having one to three nitrogen atoms, or one or two nitrogen atoms and one oxygen or sulfur, and said ring substituted by one or two ($C_1$-$C_4$)alkyl or phenyl, or condensed with benzo, or substitued by one of pyridyl, furyl or thienyl, said phenyl or benzo optionally substituted by one of iodo or trifluoromethylthio, or one or two of fluoro, chloro, bromo, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl, or trifluoromethyl, and said pyridyl, furyl or thienyl optionally substituted in the 3-position by fluoro, chloro, ($C_1$-$C_4$)alkyl or ($C_1$-$C_4$)alkoxy;

oxazole or thiazole condensed with a 6-membered aromatic group containing one or two nitrogen atoms, with thiophene or with furane, each optionally substituted by one of fluoro, chloro, bromo, trifluoromethyl, methylthio or methylsulfinyl;

imidazolopyridine; naphthothiazole; or naphthoxazole;

$R_3$ and $R_4$ are the same or different and are hydrogen, fluoro, chloro, bromo, trifluoromethyl, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl, or nitro, or $R_3$ and $R_4$ taken together are ($C_1$-$C_4$)alkanedioxy; and

$R_5$ is hydrogen or methyl; or

a pharmaceutically acceptable base addition salt of a compound of formula I wherein $R_1$ is hydroxy, or an acid addition salt of a compound of formula I wherein $R_1$ is di($C_1$-$C_4$)alkylamino or ($C_1$-$C_4$)alkoxy substituted by N-morpholino or di($C_1$-$C_4$)alkylamino; with the proviso that when $R_2$ is either a heterocyclic 5-membered ring having one heteroatom which is sulphur or a heterocyclic 6-membered ring having one heteroatom which is nitrogen, then said ring is either condensed with benzo, or substituted by one of pyridyl, furyl or thienyl, said benzo and said pyridyl, furyl and thienyl being optionally substituted as defined above, characterized by reacting a compound of the formula:

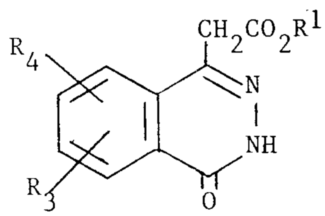

wherein $R_3$ and $R_4$ are as defined above, and R' is hydrogen, methyl or ethyl, with a compound of the formula

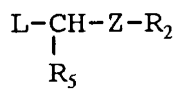

wherein L is a leaving group, and when R' is methyl or ethyl, hydrolyzing to form corresponding compounds wherein R' is hydrogen, and, if desired, converting to a pharmaceutically acceptable base addition salt by reaction with a pharmaceutically acceptable cation, and, if desired, converting $R_1$ into a conventional ester-forming residue convertible in vivo to hydroxy by alkylating the sodium salt of compound (I) wherein $R_1$ is hydroxy.

**2.** A process according to claim 1 wherein X is oxygen.

**3.** A process according to claim 1 or 2 wherein Z is a covalent bond, $R_1$ is hydroxy, and $R_3$, $R_4$ and $R_5$ are each hydrogen.

**4.** A process according to claim 1, 2 or 3 wherein $R_2$ is 5-bromo-2-benzothiazolyl, 5-fluoro-2-benzothiazolyl, 5-trifluoromethyl-2-benzothiazolyl, 5-chloro-2-benzothiazolyl, 4,5-difluoro-2-benzothiazolyl, 4,7-dichloro-2-benzothiazolyl or 5,7-dichloro-2-benzothiazolyl.

**5.** A process according to claim 1 or 2 wherein $R_3$ is hydrogen, 5-fluoro, 5-chloro, 5-bromo or 5-methyl, and $R_4$ is hydrogen; 6- or 7- substituted chloro, bromo, methyl, isopropyl, methoxy, nitro or trifluoromethyl; 4,5-difluoro, or 5,7-dichloro.

**6.** A process according to claim 1 wherein $R_5$ is methyl and L is $OSO_2R_6$ wherein $R_6$ is $(C_1\text{-}C_4)$alkyl, trifluoromethyl, or phenyl optionally substituted by methyl, chloro, bromo, or nitro.

**7.** A process according to claim 1 wherein said compound of the formula

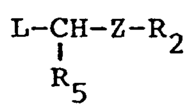

wherein L is chloro, Z is a covalent bond, $R_5$ is hydrogen and $R_2$ is oxazole or thiazole condensed with Y wherein Y is a 6-membered aromatic group containing one or two nitrogen atoms, or with thiophene or with furane, each of which optionally substituted by one of fluoro, chloro, bromo, trifluoromethyl, methylthio or methylsulfinyl; or 1,2,4-oxadiazol-3-yl or 1,2,4-thiadiazol-3-yl optionally substituted by $R_7$ wherein $R_7$ is one of iodo or trifluoromethylthio, or one or two of fluoro, chloro, bromo, $(C_1\text{-}C_4)$alkyl, $(C_1\text{-}C_4)$alkoxy, $(C_1\text{-}C_4)$alkylthio, $(C_1\text{-}C_4)$alkylsulfinyl, $(C_1\text{-}C_4)$alkylsulfonyl or trifluoromethyl, is prepared by reacting a compound of the formula

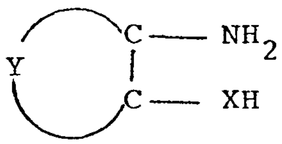

VI

or

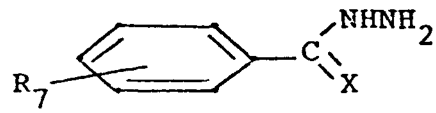

VII

wherein X is O or S, and Y and $R_7$ are as defined before, with tri(($C_1$-$C_4$)alkoxy)$CH_2Cl$.

## Revendications

**Revendications pour les Etats contractants suivants : BE, CH, DE, FR, GB, IT, LI, LU, NL, SE**

**1.** Composé de formule

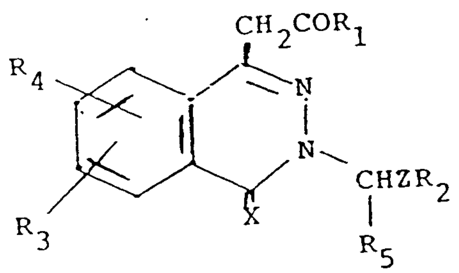

I

dans laquelle

X représente l'oxygène ou le soufre ;

Z représente une liaison covalente, O, S, un groupe NH ou $CH_2$ ;

$R_1$ représente un groupe hydroxy, ou un résidu classique de formation d'ester qui peut être transformé in vivo en un groupe hydroxy ;

$R_2$ représente un noyau pentagonal hétérocyclique ayant un atome d'azote, d'oxygène ou de soufre, deux atomes d'azote dont l'un peut être remplacé par l'oxygène ou le soufre, ou bien trois atomes d'azote dont l'un peut être remplacé par l'oxygène ou le soufre, ledit noyau étant substitué avec un ou deux groupes fluoro, chloro, alkyle en $C_1$ à $C_4$ ou phényle, ou condensé avec un groupe benzo, ou substitué avec un groupe pyridyle, furyle ou thiényle, ledit groupe phényle ou benzo étant facultativement substitué avec un groupe iodo ou trifluorométhylthio, ou bien un ou deux groupes fluoro, chloro, bromo, alkyle en $C_1$ à $C_4$, alkoxy en $C_1$ à $C_4$, alkylthio en $C_1$ à $C_4$, alkylsulfinyle en $C_1$ à $C_4$, alkylsulfonyle en $C_1$ à $C_4$ ou trifluorométhyle, et ledit groupe pyridyle, furyle ou thiényle étant facultativement substitué en position 3 avec un groupe fluoro, chloro, bromo, alkyle en $C_1$ à $C_4$ ou alkoxy en $C_1$ à $C_4$ ;

un noyau hexagonal hétérocyclique ayant un à trois atomes d'azote, ou bien un ou deux atomes d'azote et un atome d'oxygène ou de soufre, ledit noyau étant substitué avec un ou deux groupes alkyle en $C_1$ à $C_4$ ou phényle, ou condensé avec un groupe benzo, ou bien substitué avec un groupe pyridyle, furyle ou thiényle, ledit groupe phényle ou benzo étant facultativement substitué avec un groupe iodo ou trifluorométhylthio, ou bien un ou deux groupes fluoro, chloro, bromo, alkyle en $C_1$ à $C_4$, alkoxy en $C_1$ à $C_4$, alkylthio en $C_1$ à $C_4$, alkylsulfinyle en $C_1$ à $C_4$, alkylsulfonyle en $C_1$ à $C_4$ ou trifluorométhyle, et ledit groupe pyridyle, furyle ou thiényle étant facultativement substitué en position 3 avec un groupe fluoro, chloro, alkyle en $C_1$ à $C_4$ ou alkoxy en $C_1$ à $C_4$ ;

un groupe oxazole ou thiazole condensé avec un groupe aromatique hexagonal contenant un ou deux atomes d'azote, avec le thiophène ou avec le furanne, chacun facultativement substitué avec un groupe fluoro, chloro, bromo, trifluorométhyle, méthylthio ou méthylsulfinyle ;

un groupe imidazolopyridine ; naphtothiazole ; ou naphtoxazole ;

$R_3$ et $R_4$ sont identiques ou différents et représentent l'hydrogène, des groupes fluoro, chloro, bromo, trifluorométhyle, alkyle en $C_1$ à $C_4$, alkoxy en $C_1$ à $C_4$, alkylthio en $C_1$ à $C_4$, alkylsulfinyle en $C_1$ à $C_4$, alkylsulfonyle en $C_1$ à $C_4$ ou nitro, ou bien $R_3$ et $R_4$, pris conjointement, représentent un groupe alcanedioxy en $C_1$ à $C_4$ ; et $R_5$ représente l'hydrogène ou un groupe méthyle ; ou

un sel d'addition de base pharmaceutiquement acceptable d'un composé de formule I dans laquelle $R_1$ représente un groupe hydroxy, ou un sel d'addition d'acide d'un composé de formule I

dans laquelle $R_1$ représente un groupe di(alkylamino en $C_1$ à $C_4$) ou alkoxy en $C_1$ à $C_4$ substitué avec un groupe N-morpholino ou di(alkylamino en $C_1$ à $C_4$) ;

sous réserve que, lorsque $R_2$ représente un noyau pentagonal hétérocyclique ayant un hétéro-atome qui est un atome de soufre ou un noyau hexagonal hétérocyclique ayant un hétéro-atome qui est un atome d'azote, ledit noyau soit alors condensé avec un groupe benzo ou bien substitué avec un groupe pyridyle, furyle ou thiényle, ledit groupe benzo et ledit groupe pyridyle, furyle ou thiényle étant facultativement substitués de la manière définie ci-dessus.

**2.** Composé suivant la revendication 1, caractérisé en ce que X représente l'oxygène.

**3.** Composé suivant la revendication 1 ou 2, caractérisé en ce que $R_2$ représente un groupe benzothiazolyle, benzoxazolyle, isoquinolyle, benzothiophényle ou benzofurannyle, ou bien oxadiazolyle ou indolyle substitué.

**4.** Composé suivant la revendication 1, caractérisé en ce que X représente l'oxygène, Z représente une liaison covalente ou un groupe $CH_2$, $R_1$ représente un groupe hydroxy, $R_2$ représente un groupe benzothiazole-2-yle, benzothiazole-5-yle, benzo-isothiazole-3-yle, benzoxazole-2-yle, 2-quinolyle, 2-quinoxalyle, oxazolo[4,5-b]pyridine-2-yle, benzothiophène-2-yle, benzofuranne-2-yle ou thiazolo[4,5-b]-pyridine-2-yle facultativement substitué, ou bien 1,2,4-oxadiazole-3-yle, 1,2,4-oxadiazole-5-yle, isothiazole-5-yle, isothiazole-4-yle, 1,3,4-oxadiazole-5-yle, 1,2,5-thiadiazole-3-yle, oxazole-2-yle, thiazole-2-yle ou thiazole-4-yle substitué, et $R_3$, $R_4$ et $R_5$ représentent l'hydrogène.

**5.** Composé suivant la revendication 1 ou 2, caractérisé en ce que $R_2$ représente un groupe 2-benzothiazolyle substitué sur le groupe benzo avec un groupe trifluorométhylthio ou bien un ou deux groupes chloro, bromo, méthyle, méthoxy, trifluorométhyle ou 6,7-benzo.

**6.** Composé suivant la revendication 1, 2 ou 3, caractérisé en ce que $R_3$ représente l'hydrogène, un groupe 5-fluoro, 5-chloro, 5-bromo ou 5-méthyle, et $R_4$ représente l'hydrogène ; ou un substituant chloro, bromo, méthyle, isopropyle, méthoxy, nitro ou trifluorométhyle en position 6 ou 7 ; un groupe 4,5-difluoro ou 5,7-dichloro.

**7.** Composé suivant la revendication 1 ou 2, caractérisé en ce que $R_2$ représente un groupe benzothiazole-2-yle ou quinoxalyle facultativement substitué, et $R_3$ et $R_4$ représentent chacun un groupe chloro.

**8.** Composé suivant la revendication 1, caractérisé en ce qu'il est sous forme du sel de sodium ou du sel de N-méthylglucamine.

**9.** Composition pharmaceutique comprenant un composé suivant l'une quelconque des revendications 1 à 8, en mélange avec un support pharmaceutiquement acceptable.

**10.** Composé suivant l'une quelconque des revendications 1 à 8, destiné à être utilisé comme médicament.

**11.** Utilisation d'un composé de formule (I) suivant l'une quelconque des revendications 1 à 8 pour la production d'un médicament destiné à être utilisé comme inhibiteur de l'aldose-réductase.

**Revendications pour les Etats contractants suivants : AT, ES, GR**

**1.** Procédé de préparation d'un composé de formule

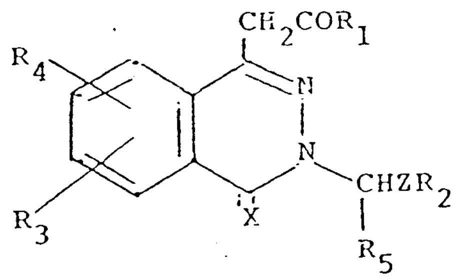

I

dans laquelle

X représente l'oxygène ou le soufre ;

Z représente une liaison covalente, O, S, un groupe NH ou $CH_2$ ;

$R_1$ représente un groupe hydroxy, ou un résidu classique de formation d'ester qui peut être transformé in vivo en un groupe hydroxy ;

$R_2$ représente un noyau pentagonal hétérocyclique ayant un atome d'azote, d'oxygène ou de soufre, deux atomes d'azote dont l'un peut être remplacé par l'oxygène ou le soufre, ou bien trois atomes d'azote dont l'un peut être remplacé par l'oxygène ou le soufre, ledit noyau étant substitué avec un ou deux groupes fluoro, chloro, alkyle en $C_1$ à $C_4$ ou phényle, ou condensé avec un groupe benzo, ou bien substitué avec un groupe pyridyle, furyle ou thiényle, ledit groupe phényle ou benzo étant facultativement substitué avec un groupe iodo ou trifluorométhylthio, ou bien un ou deux groupes fluoro, chloro, bromo, alkyle en $C_1$ à $C_4$, alkoxy en $C_1$ à $C_4$, alkylthio en $C_1$ à $C_4$, alkylsulfinyle en $C_1$ à $C_4$, alkylsulfonyle en $C_1$ à $C_4$ ou trifluorométhyle, et ledit groupe pyridyle, furyle ou thiényle étant facultativement substitué en position 3 avec un groupe fluoro, chloro, bromo, alkyle en $C_1$ à $C_4$ ou alkoxy en $C_1$ à $C_4$ ;

un noyau hexagonal hétérocyclique ayant un à trois atomes d'azote, ou bien un ou deux atomes d'azote et un atome d'oxygène ou de soufre, ledit noyau étant substitué avec un ou deux groupes alkyle en $C_1$ à $C_4$ ou phényle, ou condensé avec un groupe benzo, ou bien substitué avec un groupe pyridyle, furyle ou thiényle, ledit groupe phényle ou benzo étant facultativement substitué avec un groupe iodo ou trifluorométhylthio, ou avec un ou deux groupes fluoro, chloro, bromo, alkyle en $C_1$ à $C_4$, alkoxy en $C_1$ à $C_4$, alkylthio en $C_1$ à $C_4$, alkylsulfinyle en $C_1$ à $C_4$, alkylsulfonyle en $C_1$ à $C_4$ ou trifluorométhyle, et ledit groupe pyridyle, furyle ou thiényle étant facultativement substitué en position 3 avec un groupe fluoro, chloro, alkyle en $C_1$ à $C_4$ ou alkoxy en $C_1$ à $C_4$ ;

un groupe oxazole ou thiazole condensé avec un groupe aromatique hexagonal contenant un ou deux atomes d'azote, avec le thiophène ou avec le furanne, chacun facultativement substitué avec un groupe fluoro, chloro, bromo, trifluorométhyle, méthylthio ou méthylsulfinyle ;

un groupe imidazolopyridine ; naphtothiazole ; ou naphtoxazole ;

$R_3$ et $R_4$ sont identiques ou différents et représentent l'hydrogène, des groupes fluoro, chloro, bromo, trifluorométhyle, alkyle en $C_1$ à $C_4$, alkoxy en $C_1$ à $C_4$, alkylthio en $C_1$ à $C_4$, alkylsulfinyle en $C_1$ à $C_4$, alkylsulfonyle en $C_1$ à $C_4$ ou nitro, ou bien $R_3$ et $R_4$, pris conjointement, représentent un groupe alcanedioxy en $C_1$ à $C_4$ ; et

$R_5$ représente l'hydrogène ou un groupe méthyle ; ou

d'un sel d'addition de base pharmaceutiquement acceptable d'un composé de formule I dans laquelle $R_1$ représente un groupe hydroxy, ou d'un sel d'addition d'acide d'un composé de formule I dans laquelle $R_1$ représente un groupe di(alkylamino en $C_1$ à $C_4$) ou alkoxy en $C_1$ à $C_4$ substitué avec un groupe N-morpholino ou di(alkylamino en $C_1$ à $C_4$) ; sous réserve que, lorsque $R_2$ représente un noyau pentagonal hétérocyclique ayant un hétéro-atome qui est un atome de soufre ou un noyau hexagonal hétérocyclique ayant un hétéro-atome qui est un atome d'azote, ledit noyau soit alors condensé avec un groupe benzo ou bien substitué avec un groupe pyridyle, furyle ou thiényle, ledit groupe benzo et ledit groupe pyridyle, furyle ou thiényle étant facultativement substitué de la manière définie ci-dessus,

caractérisé en ce qu'il consiste à faire réagir un composé de formule :

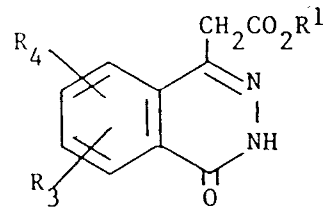

dans laquelle $R_3$ et $R_4$ répondent aux définitions précitées et R' représente l'hydrogène, un groupe méthyle ou éthyle, avec un composé de formule

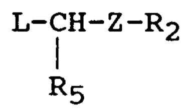

dans laquelle L représente un groupe partant et, lorsque R' représente un groupe méthyle ou éthyle, à effectuer une hydrolyse pour former le composé correspondant dans lequel R' représente l'hydrogène, et, si cela est désiré, à effectuer une transformation en un sel d'addition de base pharmaceutiquement acceptable par réaction avec un cation pharmaceutiquement acceptable, et, si cela est désiré, à transformer $R_1$ en un résidu classique de formation d'ester, pouvant être transformé in vivo en un groupe hydroxy par alkylation du sel de sodium du composé de formule (I) dans laquelle $R_1$ représente un groupe hydroxy.

**2.** Procédé suivant la revendication 1, dans lequel X représente l'oxygène.

**3.** Procédé suivant la revendication 1 ou 2, dans lequel Z représente une liaison covalente, $R_1$ représente un groupe hydroxy, et $R_3$, $R_4$ et $R_5$ représentent chacun l'hydrogène.

**4.** Procédé suivant la revendication 1, 2 ou 3, dans lequel $R_2$ représente un groupe 5-bromo-2-benzothiazolyle, 5-fluoro-2-benzothiazolyle, 5-trifluorométhyl-2-benzothiazolyle, 5-chloro-2-benzothiazolyle, 4,5-difluoro-2-benzothiazolyle, 4,7-dichloro-2-benzothiazolyle ou 5,7-dichloro-2-benzothiazolyle.

**5.** Procédé suivant la revendication 1 ou 2, dans lequel $R_3$ représente l'hydrogène , un groupe 5-fluoro, 5-chloro, 5-bromo ou 5-méthyle, et $R_4$ représente l'hydrogène ; ou un substituant chloro, bromo, méthyle, isopropyle, méthoxy, nitro ou trifluorométhyle en position 6 ou 7 ; un groupe 4,5-difluoro ou 5,7-dichloro.

**6.** Procédé suivant la revendication 1, dans lequel $R_5$ représente un groupe méthyle et L représente un groupe $OSO_2R_6$ dans lequel $R_6$ représente un groupe alkyle en $C_1$ à $C_4$, trifluorométhyle, ou phényle facultativement substitué avec un groupe méthyle, chloro, bromo ou nitro.

**7.** Procédé suivant la revendication 1, dans lequel le composé de formule

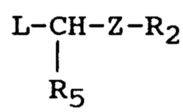

dans laquelle L représente un groupe chloro, Z représente une liaison covalente, $R_5$ représente l'hydrogène et $R_2$ représente un groupe oxazole ou thiazole condensé avec Y, Y étant un groupe aromatique hexagonal contenant un ou deux atomes d'azote, ou avec le thiophène ou bien avec le furanne, chacun de ces groupes étant facultativement substitué avec un groupe fluoro, chloro, bromo, trifluorométhyle, méthylthio ou méthylsulfinyle ; ou 1,2,4-oxadiazole-3-yle ou 1,2,4-thiadiazole-3-yle facultativement substitué avec $R_7$, $R_7$ représentant un groupe iodo ou trifluorométhylthio, ou bien un ou deux groupes fluoro, chloro, bromo, alkyle en $C_1$ à $C_4$, alkoxy en $C_1$ à $C_4$, alkylthio en $C_1$ à $C_4$,

alkylsulfinyle en $C_1$ à $C_4$, alkylsulfonyle en $C_1$ à $C_4$ ou trifluorométhyle, est préparé par réaction d'un composé de formule

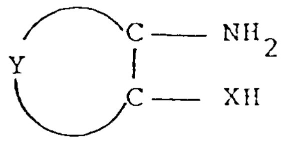

VI

ou

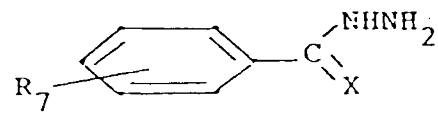

VII

dans laquelle X représente O ou S, et Y et $R_7$ répondent aux définitions précitées, avec un composé de formule tri(alkoxy en $C_1$ à $C_4$)$CH_2Cl$.

**Patentansprüche**
**Patentansprüche für folgende Vertragsstaaten : BE, CH, DE, FR, GB, IT, LI, LU, NL, SE**

**1.** Verbindung der Formel

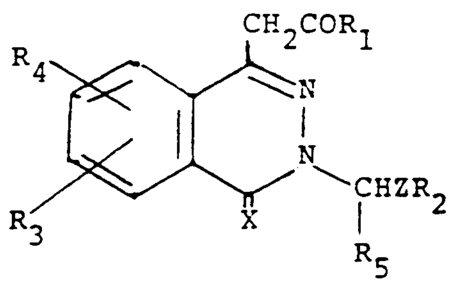

I

in welcher

X Sauerstoff oder Schwefel ist;

Z eine kovalente Bindung, O, S, NH oder $CH_2$ ist,

$R_1$ eine Hydroxygruppe oder ein üblicher esterbildender Rest ist, der in vivo in eine Hydroxygruppe umwandelbar ist;

$R_2$ ein heteroyclischer 5-gliedriger Ring mit einem Stickstoff-, Sauerstoff- oder Schwefelatom, 2 Stickstoffatomen, von welchen eines durch Sauerstoff oder Schwefel ersetzt sein kann, oder 3 Stickstoffatomen, von welchen eines durch Sauerstoff oder Schwefel ersetzt sein kann, wobei der Ring durch 1 oder 2 Fluor-, Chlor-, ($C_1$-$C_4$)-Alkyl- oder Phenylgruppen substituiert oder mit Benzo kondensiert oder durch eine Pyridyl-, Furyl- oder Thienylgruppe substituiert sein kann, wobei Phenyl oder Benzo gegebenenfalls durch eine Jod- oder Trifluormethylthiogruppe oder durch 1 oder 2 Fluor-, Chlor-, Brom-, ($C_1$-$C_4$)-Alkyl-, ($C_1$-$C_4$)-Alkoxy-, ($C_1$-$C_4$)-Alkylthio-, ($C_1$-$C_4$)-Alkylsulfinyl-, ($C_1$-$C_4$)-Alkylsulfonyl- oder Trifluormethylgruppen substituiert sein kann und wobei Pyridyl, Furyl oder Thienyl gegebenenfalls in der 3-Stellung durch Fluor, Chlor, Brom, ($C_1$-$C_4$)-Alkyl oder ($C_1$-$C_4$)-Alkoxy substituiert sein kann;

ein heterocyclischer 6-gliedriger Ring mit 1 bis 3 Stickstoffatomen oder 1 oder 2 Stickstoffatomen und 1 Sauerstoff- oder Schwefelatom, wobei dieser Ring durch 1 oder 2 ($C_1$-$C_4$)-Alkyl- oder Phenylgruppen substituiert oder mit Benzo kondensiert oder durch eine Pyridyl-, Furyl- oder Thienylgruppe substituiert sein kann, wobei Phenyl oder Benzo gegebenenfalls durch eine Jod- oder Trifluormethylthiogruppe oder 1 oder 2 Fluor-, Chlor-, Brom-, ($C_1$-$C_4$)-Alkyl-, ($C_1$-$C_4$)-Alkoxy-, ($C_1$-$C_4$)-Alkylthio-, ($C_1$-$C_4$)-Alkylsulfinyl-, ($C_1$-$C_4$)-Alkylsulfonyl- oder Trifluormethylgruppen substituiert sein kann und wobei Pyridyl, Furyl oder Thienyl gegebenenfalls in der 3-Stellung durch Fluor, Chlor, ($C_1$-$C_4$)-Alkyl oder ($C_1$-$C_4$)-Alkoxy substituiert sein kann;

Oxazol oder Thiazol, kondensiert mit einer 6-gliedrigen aromatischen, 1 oder 2 Stickstoffatome enthaltenden Gruppe, mit Thiophen oder mit Furan, die jeweils gegebenenfalls durch eine

Fluor-, Chlor-, Brom-, Trifluormethyl-, Methylthio- oder Methylsulfinylgruppe substituiert sein können; Imidazolopyridin, Naphthothiazol oder, Naphthoxazol ist;
$R_3$ und $R_4$ gleich oder verschieden sind und Wasserstoff, Fluor, Chlor, Brom, Trifluormethyl, $(C_1-C_4)$-Alkyl, $(C_1-C_4)$-Alkoxy, $(C_1-C_4)$-Alkylthio, $(C_1-C_4)$-Alkylsulfinyl, $(C_1-C_4)$-Alkylsulfonyl oder Nitro sind, oder $R_3$ und $R_4$ bedeuten, zusammen genommen, $(C_1-C_4)$-Alkandioxy; und
$R_5$ Wasserstoff oder Methyl ist, oder

ein pharmazeutisch annehmbares Basen-Additionssalz einer Verbindung der Formel I, worin $R_1$ Hydroxy ist, oder ein Säure-Additionssalz einer Verbindung der Formel I, worin $R_1$ Di-$(C_1-C_4)$-alkylamino oder $(C_1-C_4)$-Alkoxy, substituiert durch N-Morpholino oder Di-$(C_1-C_4)$-alkylamino, ist,

mit der Maßgabe, daß, wenn $R_2$ entweder einen heterocyclischen 5-gliedrigen Ring mit einem Heteroatom, das Schwefel ist, oder einen heterocyclischen 6-gliedrigen Ring mit einem Heteroatom, das Stickstoff ist, bedeutet, der Ring dann entweder mit Benzo kondensiert oder durch eine Pyridyl-, Furyl-oder Thienylgruppe substituiert ist, wobei Benzo und die Pyridyl-, Furyl- und Thienylgruppe gegebenenfalls substituiert sein können, wie dies oben definiert ist.

**2.** Verbindung gemäß Anspruch 1, dadurch gekennzeichnet, daß X Sauerstoff ist.

**3.** Verbindung gemäß Anspruch 1 oder 2, dadurch gekennzeichnet, daß $R_2$ gegebenenfalls substituiertes Benzothiazolyl, Benzoxazolyl, Isochinolyl, Benzothiophen-yl oder Benzofuran-yl oder substituiertes Oxadiazolyl oder Indolyl ist.

**4.** Verbindung gemäß Anspruch 1, dadurch gekennzeichnet, daß X Sauerstoff ist, Z eine kovalente Bindung oder $CH_2$ ist, $R_1$ Hydroxy ist, $R_2$ gegebenenfalls substituiertes Benzothiazol-2-yl, Benzothiazol-5-yl, Benzoisothiazol-3-yl, Benzoxazol-2-yl, 2-Chinolyl, 2-Chinoxalyl, Oxazolo[4,5b]pyridin-2-yl, Benzothiophen-2-yl, Benzofuran-2-yl oder Thiazolo[4,5-b]pyridin-2-yl oder substituiertes 1,2,4-Oxadiazol-3-yl, 1,2,4-Oxadiazol-5-yl, Isothiazol-5-yl, Isothiazol-4-yl, 1,3,4-Oxadiazol-5-yl, 1,2,5-Thiadiazol-3-yl, Oxazol-2-yl, Thiazol-2-yl oder Thiazol-4-yl ist und $R_3$, $R_4$ und $R_5$ Wasserstoff bedeuten.

**5.** Verbindung gemäß Anspruch 1 oder 2, dadurch gekennzeichnet, daß $R_2$ 2-Benzothiazolyl bedeutet, in der Benzogruppe substituiert durch eine Trifluormethylthiogruppe oder eine oder zwei Chlor-, Brom-, Methyl-, Methoxy-, Trifluormethyl- oder 6,7-Benzogruppen.

**6.** Verbindung gemäß Anspruch 1, 2 oder 3, dadurch gekennzeichnet, daß $R_3$ Wasserstoff, 5-Fluor, 5-Chlor, 5-Brom oder 5-Methyl bedeutet und $R_4$ Wasserstoff, 6- oder 7-substituiertes Chlor, Brom, Methyl, Isopropyl, Methoxy, Nitro oder Trifluormethyl, 4,5-Difluor oder 5,7-Dichlor bedeutet.

**7.** Verbindung gemäß Anspruch 1 oder 2, dadurch gekennzeichnet, daß $R_2$ gegebenenfalls substituiertes Benzothiazol-2-yl oder Chinoxalyl bedeutet ist und $R_3$ und $R_4$ jeweils Chlor bedeuten.

**8.** Verbindung gemäß Anspruch 1, dadurch gekennzeichnet, daß sie in Form des Natriumsalzes oder des N-Methylglucaminsalzes vorliegt.

**9.** Pharmazeutische Zusammensetzung, die eine Verbindung gemäß irgendeinem der Ansprüche 1 bis 8 in Mischung mit einem pharmazeutisch annehmbaren Träger umfaßt.

**10.** Verbindung gemäß irgendeinem der Ansprüche 1 bis 8 zur Verwendung als Arzneimittel.

**11.** Die Verwendung einer Verbindung der Formel I gemäß irgendeinem der Ansprüche 1 bis 8 für die Herstellung eines Arzneimittels zur Verwendung als Aldosereduktase-Inhibitor.

**Patentansprüche für folgende Vertragsstaaten : AT, ES, GR**

**1.** Verfahren zur Herstellung einer Verbindung der Formel

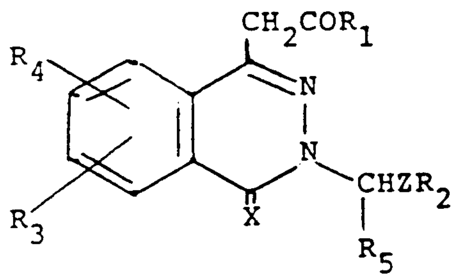

I

in welcher

X Sauerstoff oder Schwefel ist;

Z eine kovalente Bindung, O, S, NH oder $CH_2$ ist,

$R_1$ eine Hydroxygruppe oder ein üblicher esterbildender Rest ist, der in vivo in eine Hydroxygruppe umwandelbar ist;

$R_2$ ein heteroyclischer 5-gliedriger Ring mit einem Stickstoff-, Sauerstoff- oder Schwefelatom, 2 Stickstoffatomen, von welchen eines durch Sauerstoff oder Schwefel ersetzt sein kann, oder 3 Stickstoffatomen, von welchen eines durch Sauerstoff oder Schwefel ersetzt sein kann, wobei der Ring durch 1 oder 2 Fluor-, Chlor-, ($C_1$-$C_4$)-Alkyl- oder Phenylgruppen substituiert oder mit Benzo kondensiert oder durch eine Pyridyl-, Furyl- oder Thienylgruppe substituiert sein kann, wobei Phenyl oder Benzo gegebenenfalls durch eine Jod- oder Trifluormethylthiogruppe oder durch 1 oder 2 Fluor-, Chlor-, Brom-, ($C_1$-$C_4$)-Alkyl-, ($C_1$-$C_4$)-Alkoxy-, ($C_1$-$C_4$)-Alkylthio-, ($C_1$-$C_4$)-Alkylsulfinyl-, ($C_1$-$C_4$)-Alkylsulfonyl- oder Trifluormethylgruppen substituiert sein kann und wobei Pyridyl, Furyl oder Thienyl gegebenenfalls in der 3-Stellung durch Fluor, Chlor, Brom, ($C_1$-$C_4$)-Alkyl oder ($C_1$-$C_4$)-Alkoxy substituiert sein kann;

ein heterocyclischer 6-gliedriger Ring mit 1 bis 3 Stickstoffatomen oder 1 oder 2 Stickstoffatomen und 1 Sauerstoff- oder Schwefelatom, wobei dieser Ring durch 1 oder 2 ($C_1$-$C_4$)-Alkyl- oder Phenylgruppen substituiert oder mit Benzo kondensiert oder durch eine Pyridyl-, Furyl- oder Thienylgruppe substituiert sein kann, wobei Phenyl oder Benzo gegebenenfalls durch eine Jod- oder Trifluormethylthiogruppe oder 1 oder 2 Fluor-, Chlor-, Brom-, ($C_1$-$C_4$)-Alkyl-, ($C_1$-$C_4$)-Alkoxy-, ($C_1$-$C_4$)-Alkylthio-, ($C_1$-$C_4$)-Alkylsulfinyl-, ($C_1$-$C_4$)-Alkylsulfonyl- oder Trifluormethylgruppen substituiert sein kann und wobei Tyridyl, Furyl oder Thienyl gegebenenfalls in der 3-Stellung durch Fluor, Chlor, ($C_1$-$C_4$)-Alkyl oder ($C_1$-$C_4$)-Alkoxy substituiert sein kann;

Oxazol oder Thiazol, kondensiert mit einer 6-gliedrigen aromatischen, 1 oder 2 Stickstoffatome enthaltenden Gruppe, mit Thiophen oder mit Furan, die jeweils gegebenenfalls durch eine Fluor-, Chlor-, Brom-, Trifluormethyl-, Methylthio- oder Methylsulfinylgruppe substituiert sein können; Imidazolopyridin, Naphthothiazol oder Naphthoxazol ist;

$R_3$ und $R_4$ gleich oder verschieden sind und Wasserstoff, Fluor, Chlor, Brom, Trifluormethyl, ($C_1$-$C_4$)-Alkyl, ($C_1$-$C_4$)-Alkoxy, ($C_1$-$C_4$)-Alkylthio, ($C_1$-$C_4$)-Alkylsulfinyl, ($C_1$-$C_4$)-Alkylsulfonyl oder Nitro sind, oder $R_3$ und $R_4$ bedeuten, zusammen genommen, ($C_1$-$C_4$)-Alkandioxy; und

$R_5$ Wasserstoff oder Methyl ist, oder

eines pharmazeutisch annehmbaren Basen-Additionssalzes einer Verbindung der Formel I, in welcher $R_1$ Hydroxy ist, oder eines Säure-Additionssalzes einer Verbindung der Formel I, in welcher $R_1$ Di-($C_1$-$C_4$)-alkylamino oder ($C_1$-$C_4$)-Alkoxy, substituiert durch N-Morpholino oder Di-($C_1$-$C_4$)-Alkylamino, ist; mit der Maßgabe, daß, wenn $R_2$ entweder einen heterocyclischen 5-gliedrigen Ring mit einem Heteroatom, das Schwefel ist, oder einen heterocyclischen 6-gliedrigen Ring mit einem Heteroatom, das Stickstoff ist, bedeutet, der Ring dann entweder mit Benzo kondensiert oder durch eine Pyridyl-, Furyl-oder Thienylgruppe substituiert ist, wobei Benzo und die Pyridyl-, Furyl- oder Thienylgruppe gegebenenfalls substituiert sein können, wie dies oben definiert ist, dadurch gekennzeichnet, daß man eine Verbindung der Formel

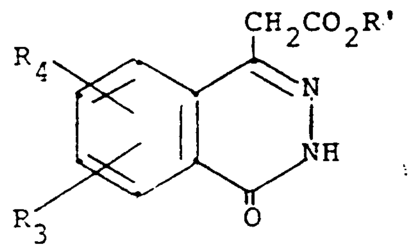

in welcher $R_3$ und $R_4$ wie oben definiert sind und R' Wasserstoff, Methyl oder Ethyl ist, mit einer Verbindung der Formel

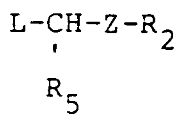

in welcher L eine sich abtrennende Gruppe ist, umsetzt, und, wenn R' Methyl oder Ethyl ist, unter Bildung entsprechender Verbindungen, in welchen R' Wasserstoff ist, hydrolysiert und, gegebenenfalls, durch Reaktion mit einem pharmazeutisch annehmbaren Kation in ein pharmazeutisch annehmbares Basen-Additionssalz umwandelt und, gegebenenfalls, $R_1$ durch Alkylieren des Natriumsalzes der Verbindung (I), in welcher $R_1$ Hydroxy ist, in einer üblichen esterbildenden Rest umwandelt, der in vivo in eine Hydroxygruppe umwandelbar ist.

**2.** Verfahren nach Anspruch 1, bei dem X Sauerstoff ist.

**3.** Verfahren nach Anspruch 1 oder 2, bei dem Z eine kovalente Bindung ist, $R_1$ Hydroxy ist und $R_3$, $R_3$ und $R_5$ jeweils Wasserstoff sind.

**4.** Verfahren nach Anspruch 1, 2 oder 3, bei dem $R_2$ 5-Brom-2-benzothiazolyl, 5-Fluor-2-benzothiazolyl, 5-Trifluormethyl-2-benzothiazolyl, 5-Chlor-2-benzothiazolyl, 4,5-Difluor-2-benzothiazolyl, 4,7-Dichlor-2-benzothiazolyl oder 5,7-Dichlor-2-benzothiazolyl ist.

**5.** Verfahren nach Anspruch 1 oder 2, bei dem $R_3$ Wasserstoff, 5-Fluor, 5-Chlor, 5-Brom oder 5-Methyl ist und $R_4$ Wasserstoff, eine 6- oder 7-substituierte Chlor-, Brom-, Methyl-, Isopropyl-, Methoxy-, Nitro- oder Trifluormethylgruppe, 4,5-Difluor oder 5,7-Dichlor ist.

**6.** Verfahren nach Anspruch 1, bei dem $R_5$ Methyl ist und L $OSO_2R_6$ ist, wobei $R_6$ $(C_1\text{-}C_4)$-Alkyl, Trifluormethyl oder Phenyl, gegebenenfalls durch Methyl, Chlor, Brom oder Nitro substituiert, ist.

**7.** Verfahren nach Anspruch 1, bei dem man eine Verbindung der Formel

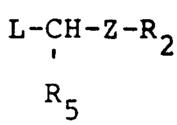

herstellt, in welcher L Chlor ist, Z eine kovalente Bindung ist, $R_5$ Wasserstoff ist und $R_2$ Oxazol oder Thiazol, kondensiert mit Y, wobei Y eine 6-gliedrige aromatische, 1 oder 2 Stickstoffatome enthaltende Gruppe ist, oder mit Thiophen oder mit Furan, die jeweils gegebenenfalls durch eine Fluor-, Chlor-, Brom-, Trifluormethyl-, Methylthio- oder Methylsulfinylgruppe substituiert sein können, oder 1,2,4-Oxadiazol-3-yl oder 1,2,4-Thiadiazol-3-yl, gegebenenfalls durch $R_7$ substituiert, wobei $R_7$ eine Jod- oder Trifluormethylthiogruppe oder 1 oder 2 Fluor-, Chlor-, Brom-, $(C_1\text{-}C_4)$-Alkyl-, $(C_1\text{-}C_4)$-Alkoxy-, $(C_1\text{-}C_4)$-Alkylthio-, $(C_1\text{-}C_4)$-Alkylsulfinyl-, $(C_1\text{-}C_4)$-Alkylsulfonyl- oder Trifluormethylgruppen bedeutet, ist, indem man eine Verbindung der Formel

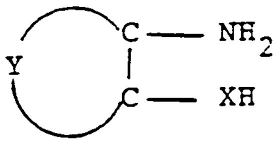

VI

oder

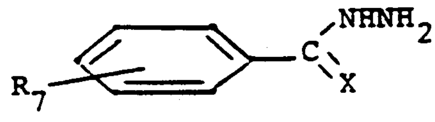

VII

in welchen X O oder S ist und Y und $R_7$ wie oben definiert sind, mit Tri-(($C_1$-$C_4$)-alkoxy)$CH_2Cl$ umsetzt.